(19) Europäisches Patentamt
European Patent Office
Office européen des brevets

(11) EP 3 530 652 A1

(12) EUROPEAN PATENT APPLICATION
published in accordance with Art. 153(4) EPC

(43) Date of publication:
**28.08.2019 Bulletin 2019/35**

(21) Application number: **17862847.5**

(22) Date of filing: **19.10.2017**

(51) Int Cl.:
***C07D 209/48*** *(2006.01)* ***A61K 31/4035*** *(2006.01)*
***A61P 35/00*** *(2006.01)*

(86) International application number:
**PCT/JP2017/037906**

(87) International publication number:
**WO 2018/074563 (26.04.2018 Gazette 2018/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.10.2016 JP 2016207402**

(71) Applicants:
- **Keio University**
  **Tokyo 108-8345 (JP)**
- **IDAC Theranostics, Inc.**
  **Bunkyo-ku**
  **Tokyo 113-0033 (JP)**

(72) Inventors:
- **HATTORI, Yutaka**
  **Tokyo 105-8512 (JP)**
- **MATSUSHITA, Maiko**
  **Tokyo 105-8512 (JP)**
- **ICHIKAWA, Daiju**
  **Tokyo 105-8512 (JP)**
- **AIDA, Shuji**
  **Tokyo 105-8512 (JP)**
- **SUGAI, Takeshi**
  **Tokyo 105-8512 (JP)**
- **YAMADA Taketo**
  **Iruma-gun**
  **Saitama 350-0495 (JP)**
- **YANAGAWA, Hiroshi**
  **Tokyo 113-0033 (JP)**
- **OGAWA, Yoko**
  **Tokyo 113-0033 (JP)**
- **TABATA, Noriko**
  **Tokyo 113-0033 (JP)**
- **YONEMURA, Yuko**
  **Tokyo 113-0033 (JP)**

(74) Representative: **Vigand, Philippe et al**
**Novagraaf International SA**
**Chemin de l'Echo 3**
**1213 Onex - Genève (CH)**

# (54) MODIFIED PHENYLPHTHALIMIDE AND PHARMACEUTICAL COMPOSITION HAVING SAME AS ACTIVE INGREDIENT

(57) A phenylphthalimide derivative represented by the following general formula is provided as a phenylphthalimide derivative useful against various cancers including multiple myeloma,

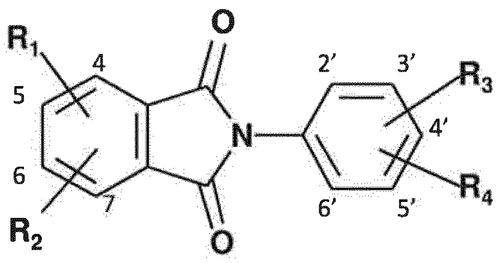

wherein $R_1$ is a group containing PEG or a hydroxy $C_{1-5}$ alkoxy group binding to position 4 or position 5, $R_2$ is an amino group at position 6, and $R_3$ and $R_4$ are each independently a hydrogen atom, an alkyl group, an aryl group, an aralkyl group, or an aryloxy group.

EP 3 530 652 A1

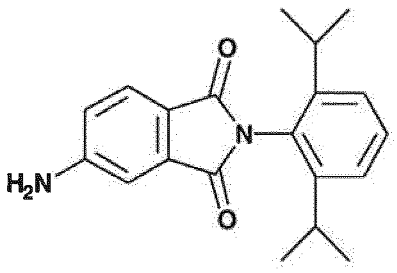

TC11

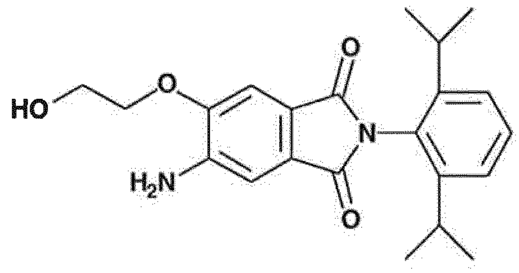

HOEtO-TC11

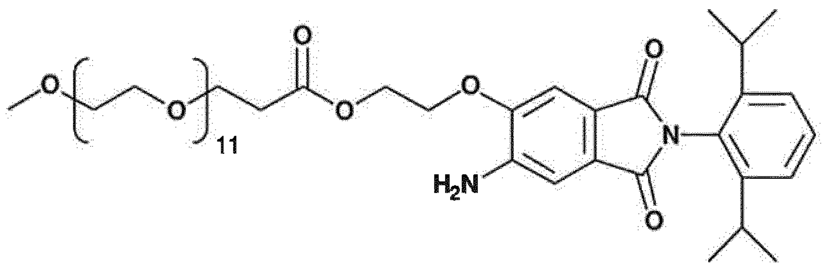

PEG11(E)-TC11

Fig. 1

## Description

TECHNICAL FIELD

The present invention relates to a novel phenylphthalimide derivative and a pharmaceutical composition containing the same as an active ingredient. The present invention more specifically relates to a phenylphthalimide derivative prepared by modification of phenylphthalimide with polyethylene glycol (PEG) or a hydroxy-alkoxy group, a method for producing the same, and a pharmaceutical composition such as an anticancer agent comprising the phenylphthalimide derivative.

BACKGROUND ART

Multiple myeloma (hereafter referred to as "MM") accounting for about 10% of hematologic cancers is one of blood disorders with poor prognosis, hematologic malignancy, which is characterized by monoclonal abnormal proliferation of B-cells (plasma cells) having undergone terminal differentiation. MM causes the presentation of various characteristic symptoms including osteolytic lesion, hematopoietic injury (particularly, anemia), hypercalcemia, renal dysfunction, immunodeficiency, and extramedullary plasmacytoma, etc. The number of national patients with MM is estimated to be 14,000, approximately 4,000 new MM patients are diagnosed annually, and annual death number is 4,066. MM is common in elderly people. Our country will be a super-aging society and thus the number of MM patients will steadily increase. Worldwide, the number of MM patients reaches as high as 750,000.

Whereas in recent years many malignant lymphoma and leukemia cases among hematologic malignancies are cured, many MM and myelodysplastic syndrome (MDS) cases are often determined to be ineligible for transplantation since MM is common in the elderly and remains as a difficult to cure disease. After specified as a teratogenic substance, thalidomide was forced to withdraw from the market as a drug. However, after the report of data demonstrating that thalidomide is effective against MM and the following clinical trials under strict limitations for use, the efficacy of thalidomide has been recognized (Non-patent document 1; Non-patent document 2). Protein cereblon (cereblon; CRBN) that binds as teratogen to thalidomide has been recently identified (Non-patent document 3). Furthermore, compounds analogous to thalidomide (lenalidomide, pomalidomide) referred to as immunomodulatory drugs (IMiDs) have emerged and been used for countermeasures against MM, a refractory disease, (Non-patent document 4; Non-patent document 5; Non-patent document 6). However, all of these cases will be resistant to these novel drugs within several years, and most cases will be fatal. In particular, the prognosis of high-risk myeloma with chromosome 13 deletion (del13), t(4;14) translocation or chromosome 17 deletion (del17) is poor, and particularly cases with deletion of chromosome 17 (del17) on which TP53 gene is present always results in poor prognosis even when novel drugs are used (Non-patent document 7; Non-patent document 8; Non-patent document 9). Subsequently, a proteasome inhibitor, bortezomib (Non-patent document 10), or carfilzomib (Non-patent document 11) has also been developed. Furthermore, drugs with other mechanisms (anti-CD38; daratumumab) include drugs, the efficacy of which is confirmed by the use thereof as a single drug (Non-patent document 12) or the same in combination with lenalidomide. Moreover, an anti-CD38 antibody (daratumumab) and an anti-PD-Ll antibody (atezolizumab) have been administered in combination. However, such antibody medicines are expensive and some of these medicines are injections and are unable to be applied via oral medication, and thus cannot always be optimum in view of patients' convenience.

To move beyond the present circumstances, a novel compound effective for even a case with high-risk chromosomal abnormality should be searched for, and the prognosis should be improved. The present inventors have synthesized to date many phthalimide derivatives, and discovered a compound TC11 (2-(2,6-diisopropylphenyl)-5-amino-1H-isoindole-1,3-dione; Fig. 1) having a high drug effect using as an index the inhibition of the proliferation of many myeloma cell line panels including high-risk myeloma cell lines that the present inventors had established themselves (Non-patent document 13; Patent document 1). TC11 comprises a phthalimide ring and a diisopropylphenyl group as the backbone, having an amino group at position 5 of the phthalimide ring (Non-patent document 14; Non-patent document 15; Patent document 2). On the other hand, TC11 lacks a glutarimide ring that is a CRBN binding site of thalidomide, lenalidomide or the like (Non-patent document 6). Nucleophosmin 1 (NPM1/B23.1) and α-tubulin being located as a target protein of TC11 in centrosomes during mitotic phase and having a function of controlling centrosome duplication have been identified (Non-patent document 11). It has been revealed that when tumor cells are treated with TC11, TC11 inhibits centrosome clustering, and as a result, induces multi-polarization and multinucleation of mitotic cells, thereby inducing apoptosis (Non-patent document 11). Similar phenomena of apoptosis induction have also been observed in experiments of the knockdown of NPM1 by siRNA using HeLa cells. Furthermore, the present inventors have also revealed that TC11 is effective against MM resistant to lenalidomide, and thalidomide in a human MM xenograft model established using immunodeficient mice (Non-patent document 16).

PEG is a nontoxic and nonimmunogenic polymer, and has a feature such that the size or the side-chain structure can be varied depending on its application. In recent years, PEGylation of target proteins or peptides has been focused

and used as a method for improving the pharmacokinetic properties and the immunologic properties of proteins and peptides having poor stability *in vivo* or for targeting etc., with the use of the above features (Non-patent document 17).

PRIOR ART REFERENCES

PATENT DOCUMENTS

[Patent document 1] WO2010/061862A1
[Patent document 2] JP 10-109975A

NON-PATENT DOCUMENTS

[Non-patent document 1] Singhal, S. et al., N. Engl. J. Med., 341: 1565-1571, 1999
[Non-patent document 2] Hattori, Y. et al., Cancer Sci., 99, 1243-1250, 2008
[Non-patent document 3] Ito, T. et al., Science, 327: 1345-1350, 2010
[Non-patent document 4] Dimopoulos, M. et al., N. Engl. J. Med., 357: 2123-2132, 2007
[Non-patent document 5] Weber, DM. et al., N. Engl. J. Med., 357: 2133-2142, 2007
[Non-patent document 6] Chamberlain, PP. et al., Nature Struct. Mol. Biol., 21: 803-809,2014
[Non-patent document 7] Avet-Loiseau, H. et al., Blood, 109, 3489-3496, 2007
[Non-patent document 8] Avet-Loiseau, H. et al., Leukemia, 24, 623-628, 2010
[Non-patent document 9] Klein, U. et al., Cancer, 117, 2136-2144, 2011
[Non-patent document 10] Jagannath, S. et al., Leukemia: 21, 151-157, 2007
[Non-patent document 11] Siegel, DS. et al., Blood, 120: 2817-2825, 2012
[Non-patent document 12] Lokhorst, HM. et al., New Engl. J. Med., 373: 1207-1219, 2015
[Non-patent document 13] Shiheido, H. et al., PLoS One, 7: e38878, 2012
[Non-patent document 14] Shibata, Y. et al., Chem. Pharm. Bull., 44: 156-162, 1996
[Non-patent document 15] Noguchi, T. et al., Bioorg. Med. Chem. Lett., 15: 5509-5513,2005
[Non-patent document 16] Matsushita, M. et al., PLoS One, 10: e0116135, 2015
[Non-patent document 17] Pasut, G., Veronese, FM., Adv. Drug Del. Rev., 61, 1177-1188, 2009

DISCLOSURE OF THE INVENTION

PROBLEMS TO BE SOLVED BY THE INVENTION

An object of the present invention is to provide a novel phenylphthalimide derivative. Another object of the present invention is to provide a pharmaceutical composition comprising the novel derivative. Another object of the present invention is to provide a therapeutic agent for various cancers including MM and immune abnormality, comprising the novel derivative.

MEANS FOR SOLVING THE PROBLEMS

TC11 has been problematic in its low water solubility in view of development thereof as a drug. It has been observed that in a tumor-transplanted mouse model, most TC11 administered via intraperitoneal injection remains unabsorbed within the abdominal cavity. Hence, TC11 should have improved water solubility upon its formulation. First, in an experiment for improving water solubility of TC11, salt formation of amino groups bound to a phthalimide ring with hydrochloric acid and maleic acid was attempted, but almost no salt formation of TC11 was confirmed. Therefore, through modification with PEG, hydroxyalkoxy, sugars, and carbohydrate, etc., the present inventors aimed at improving the water solubility, blood kinetics and drug effect of TC 11.

As a result of such trial and error, the present inventors discovered a phenylphthalimide derivative, which can be easily synthesized, and has significantly improved water solubility and blood kinetics, low toxicity, no side effect such as teratogenicity, and a high drug effect against various cancers including MM with high-risk chromosomal abnormality, and immune abnormality, and thus completed the present invention.

The present invention provides a phenylphthalimide derivative represented by the following general formula.

[Chemical formula 1]

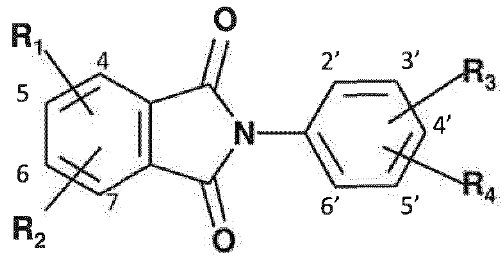

In the formula, $R_1$ is a group containing PEG or a hydroxy $C_{1-5}$ alkoxy group binding to position 4 or 5, $R_2$ is an amino group at position 6, and $R_3$ and $R_4$ are each independently a hydrogen atom, an alkyl group, an aryl group, an aralkyl group, or an aryloxy group.

$R_1$ is preferably a group containing PEG, and is further preferably a group in which PEG binds to a phthalimide ring via an ester bond. $R_1$ may also be a hydroxy $C_{1-5}$ alkoxy group. Furthermore, $R_1$ preferably binds to position 5. $R_3$ and $R_4$ are both isopropyl groups preferably located at position 2' and position 6'.

Furthermore, the present invention provides a pharmaceutical composition comprising the above derivative. The pharmaceutical composition is preferably an anticancer agent. The anticancer agent is preferably used for multiple myeloma.

EFFECT OF THE INVENTION

The derivative of the present invention can be easily synthesized, and has significantly improved water solubility and blood kinetics, low toxicity, no side effect such as teratogenicity, and a high drug effect on various cancers including MM with high-risk chromosomal abnormality and immune abnormality, and thus is advantageous as a pharmaceutical product.

BRIEF DESCRIPTION OF THE DRAWINGS

Fig. 1 shows the chemical structures of TC11, HOEtO-TC11 and PEG11(E)-TC11.
Fig. 2 shows the stability of PEG11(E)-TC11 (A) and PEG11(0)-TC11 (B) in mouse blood.
Fig. 3 shows the stability of PEG11(E)-TC11 in mouse plasma (A) and medium (B).
Fig. 4 shows the concentration dependency of *in vitro* cell proliferation inhibitory activity of TC11 and its derivatives, PEG11(E)-TC11 and HOEtO-TC11 against a high-risk MM cell line (KMS21).
Fig. 5 shows the *in vitro* cell proliferation inhibitory activity of PEG 11(E)-TC11 (A) and lenalidomide (B) against various MM cells.
Fig. 6 shows the *in vitro* cell proliferation inhibitory activity of PEG11(E)-TC11 and TC11 against high-risk MM cells (KMS34) and normal cells (WT) derived from bone marrow stromal cells.
Fig. 7 shows apoptosis induction in high-risk MM cells (MUM24) by TC11 and PEG11(E)-TC11.
Fig. 8 shows the effect of TC11 and PEG11(E)-TC11 on cell cycle.
Fig. 9A shows a pull-down experiment (A) concerning interaction of protein cereblon (CRBN) with thalidomide and TC11 (electrophoretic image).
Fig. 9B shows surface plasmon resonance biosensorgrams (B) showing the interaction of CRBN with thalidomide and lenalidomide.
Fig. 9C shows surface plasmon resonance biosensorgrams (C) showing the interaction of CRBN with TC11 and its derivatives, PEG11(E)-TC11, PEG11(O)-TC11, and HOEtO-TC11.
Fig. 10 shows mouse blood kinetics upon single intraperitoneal administration of TC11(A) and PEG11(E)-TC11(B) to mice.
Fig. 11 shows the antitumor effect of TC11, PEG11(E)-TC11, and PEG11(0)-TC11 on KMS11 cancer-bearing mice.
Fig. 12 shows a comparison of TC11, HOEtO-TC11, and lenalidomide for the antitumor effect on KMS11 cancer-bearing mice.

BEST MODE FOR CARRYING OUT THE INVENTION

TC11 is known to have an anticancer effect and a high drug effect particularly on myeloma (Non-patent document 13). Nucleophosmin 1 (NPM1/B23.1) and α-tubulin being located in centrosomes during mitotic phase as a target protein of TC11 and having a function of controlling centrosome duplication have been identified (Non-patent document 13). It has been revealed that when tumor cells are treated with TC11, TC11 inhibits centrosomal clustering, as a result, induces multi-polarization and further multinucleation of mitotic cells, thereby inducing apoptosis (Non-patent document 13). Similar phenomena of apoptosis induction have also been observed in experiments of the knockdown of NPM1 by siRNA using HeLa cells. Furthermore, the relationship between TC11 and phosphorylation of NPM1 has been studied, revealing that the phosphorylation of Ser4, Thr95, and Thr199 of NPM1 is accelerated over time by TC11. One of phosphotransferases ofNPM1 is Cyclin-dependent kinase 1 (CDK1). Acceleration of NPM1 phosphorylation by TC11 is suppressed through treatment with an inhibitor of CDK1, CGP74514A, revealing the involvement of CDK1 in acceleration of NPM1 phosphorylation.

Meanwhile, it has been revealed by a Blue native PAGE method that TC11 inhibits oligomerization of NPM1. NPM1 is considered to exhibit cell biological functions via oligomerization, while TC11 is considered to suppress the functions. Moreover, TC11 inhibits α-tubulin polymerization. It is thus inferred that mitotic catastrophe is induced by such inhibition. As described above, it is considered that TC11 induces apoptosis of myeloma cells with various mechanisms including (1) inhibition of NPM1 oligomerization, (2) inhibition of α-tubulin polymerization, followed by the suppression of TC11-induced apoptosis by CGP74514A, and (3) induction of cell death by excessive activation of CDK1, etc.

The present inventors synthesized various derivatives resulting from modification of TC11 with polyethylene glycol (PEG), hydroxyalkoxy, sugars such as glucose, carbohydrate, and the like, and intensively searched for activity of suppressing the growth of various cancers including MM. Specifically, the present inventors examined a phenylphthalimide derivative represented by the following general formula.

[Chemical formula 2]

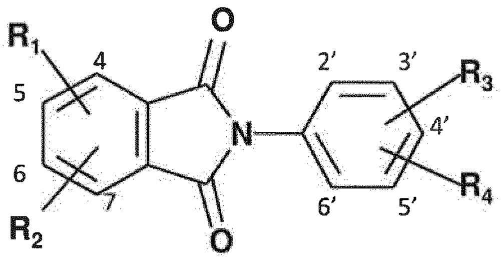

In the formula, $R_1$ contains a PEG group binding to any one of position 4, 5, 6, and 7 of a phthalimide ring via an ester group or an ether group, $R_1$ contains a hydroxy $C_{1-5}$ alkoxy group binding to any one of position 4, 5, 6, and 7 of the phthalimide ring, or, $R_1$ contains a monosaccharide, a derivative of a monosaccharide, a disaccharide, a polysaccharide, or a sugar alcohol binding to any one of position 4, 5, 6, and 7 of the phthalimide ring. $R_2$ represents an amino group, a hydroxy group, a nitro group, an O-alkyl group, or an S-alkyl group located at position 6 or 5 of the phthalimide ring. $R_3$ and $R_4$ may be the same or different, and each represents a hydrogen atom, an alkyl group, a haloalkyl group, an alkenyl group, an alkynyl group, an alkoxy group, an alkylamino group, an alkylthio group, an aralkyl group, or an aryloxy group.

The present inventors discovered that a phenylphthalimide derivative in which, in the above general formula, $R_2$ is an amino group at position 6, $R_1$ is a group containing PEG or a hydroxy $C_{1-5}$ alkoxy group binding to position 4 or 5, and $R_3$ and $R_4$ are each independently a hydrogen atom, an alkyl group or an aryl group, has an *in vitro* cancer growth-suppressing effect and *in vivo* antitumor activity as determined by the use of a xenograft model. The present inventors also discovered that special blood kinetics are observed based on the binding modes of PEG. These compounds have never been synthesized and are completely novel TC11 derivatives.

<1> Derivatives of the present invention

In the above general formula, $R_1$ is a group containing PEG or a hydroxy $C_{1-5}$ alkoxy group binding to position 4 or 5, $R_2$ is an amino group at position 6, $R_3$ and $R_4$ are each independently a hydrogen atom, an alkyl group, an aryl group, an aralkyl group, or an aryloxy group.

$R_1$ may be a group containing PEG. The term "PEG" generally refers to PEG represented by a structural formula $CH_3O(CH_2CH_2O)_nH$ wherein n is between 5 and 15 (preferably 6 and 15, further preferably 8 and 13). PEG may directly bind to a phthalimide ring via terminal oxygen atom (binding via an ether bond), or to a phthalimide ring via a divalent organic group. As such an organic group, any organic group can be used as long as it does not inhibit the anticancer activity of the derivative, and in general, polyoxyalkylene in which a non-terminal oxygen atom is substituted with an ester group (binding via an ester bond) can be used. Alkylene has a carbon number of generally 2 to 4, and a polymerization degree of generally 2 to 3. For example, in the case of polyoxyethylene, an example thereof is a $-CH_2-CH_2-C(O)-O-CH_2-CH_2-O-$ structure.

An end of PEG, which is on the side not binding to a phthalimide ring may be OH or an alkyl group having a carbon number of 1-3, and is generally a methyl group.

$R_1$ is preferably a group in which PEG binds to a phthalimide ring via polyoxyalkylene wherein a non-terminal oxygen atom is substituted with an ester group, specifically, an ester bond. Hydroxyalkoxy-TC11 is a compound generated by hydrolysis of PEG 11 (E)-TC11 by *in vivo* esterase. Accordingly, in the case of binding via an ester bond, more desirable pharmacokinetics can be exhibited as a result of *in vivo* hydrolysis. A hydroxyalkoxy derivative also exhibits an anticancer effect, and thus $R_1$ may also be a hydroxy $C_{1-5}$ alkoxy group. A hydroxy-alkoxy group has a carbon number of preferably 1 to 3, and further preferably 2.

Specific examples of $R_1$ include $CH_3O(CH_2CH_2O)_nCH_2CH_2O-$, and $CH_3O(CH_2CH_2O)_nCH_2CH_2C(O)OCH_2CH_2O-$ wherein "n" is as described above.

$R_1$ preferably binds to position 5.

$R_2$ is an amino group binding to position 6. It is considered that an amino group should be present at position 6 of a phthalimide ring for the derivative to exhibit anticancer activity. $R_1$ group is bound to position 5 or position 4 in order to avoid the inhibition of the effect of the amino group. In addition, phthalimide rings are symmetric, and thus the relationship between position 6 and position 5 or position 4 is equivalent to that between position 5 and position 6 or position 7.

$R_3$ and $R_4$ are each independently a hydrogen atom, an alkyl group, an aryl group, an aralkyl group, or an aryloxy group.

An alkyl group is generally an alkyl group having a carbon number of 1 to 6 (preferably 1 to 3). Examples thereof include a methyl group, an ethyl group, a propyl group, an isopropyl group, a butyl group, and a t-butyl group.

An aryl group is generally an aryl group having a carbon number of 6 to 10. An example thereof is a phenyl group. Aryl may be substituted with an alkyl group. The alkyl group may be similar to the above alkyl.

An aralkyl group is an aralkyl group wherein an alkyl portion has a carbon number of 1 to 6 (preferably 1 to 3). The aryl portion may be similar to the above aryl group. Examples thereof include a benzyl group, and a phenethyl group.

Alkyl portions in an alkyl group and an aralkyl group may be linear or branched.

An aryloxy group may be a group in which oxygen atoms bind to the above aryl group and aralkyl group.

The positions of $R_3$ and $R_4$ are not particularly limited. For example, $R_3$ is substituted with a phenyl group at position 2' or position 6', or $R_3$ is substituted with an oxyphenyl group at position 4'. $R_3$ and $R_4$ are both isopropyl groups, and preferably present at position 2' and position 6'.

The structure on the phenyl ring side should be hydrophobic for the derivative to exhibit anticancer activity. Accordingly, it is inferred that anticancer activity is exhibited based on the range of the above $R_3$ and $R_4$. Hence, it is also inferred that the structure on the phenyl ring side should not be PEGylated. As described later in Example 14, the structure on the phenyl ring side is also a structure that does not bind to the cereblon protein.

The derivative of the present invention can be produced through combination of known synthesis techniques. This will be described with specific examples as follows.

PEG11(E)-TC11 (Fig. 1) of the present invention can be produced by a method for producing the TC11 derivative (PEG11(E)-TC11) through PEGylation of TC11 via an ester bond, such as the following method when a reactive group for modification is mPEG 1-COOH.

[Chemical formula 3]

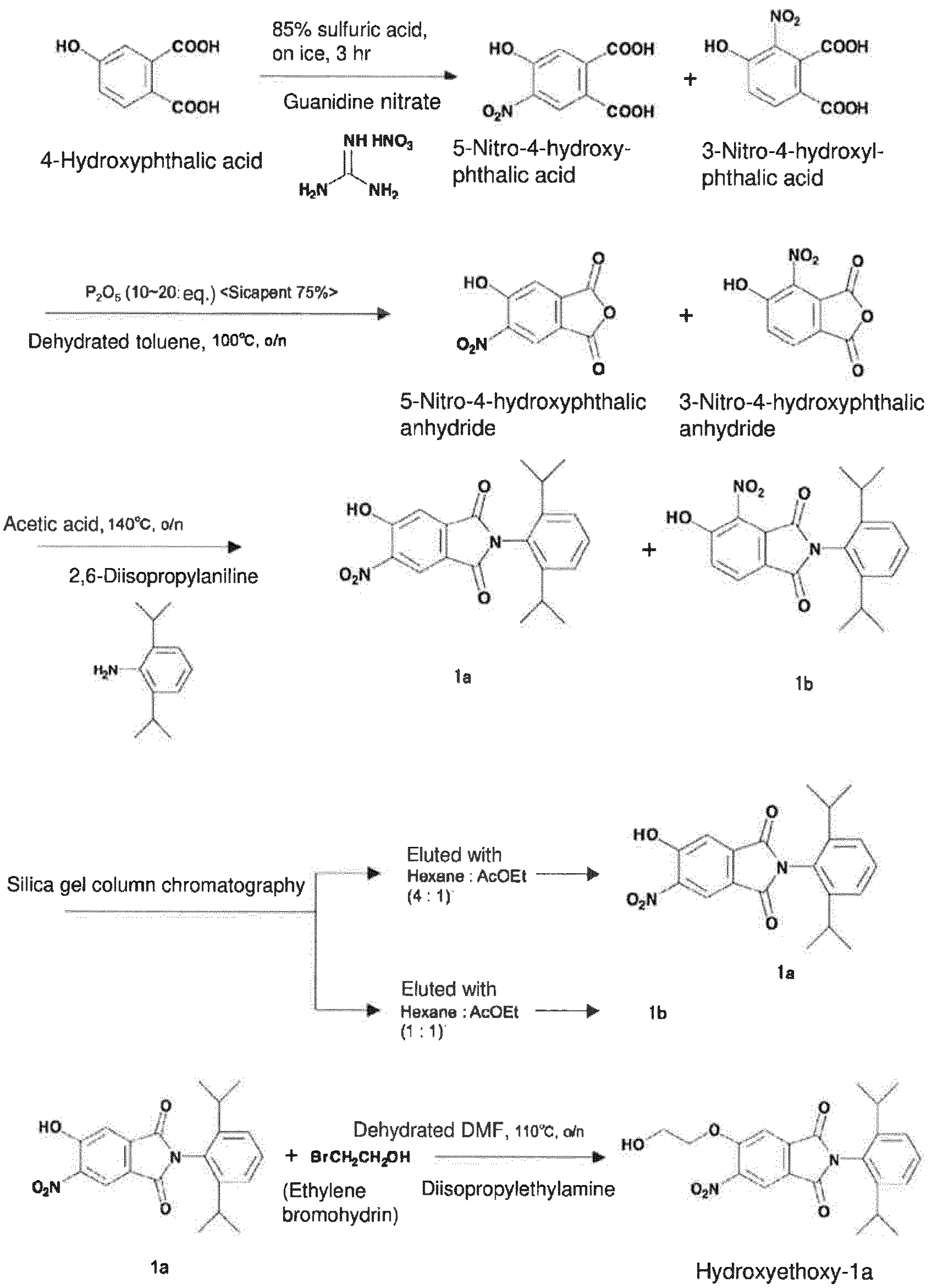


[Chemical formula 4]

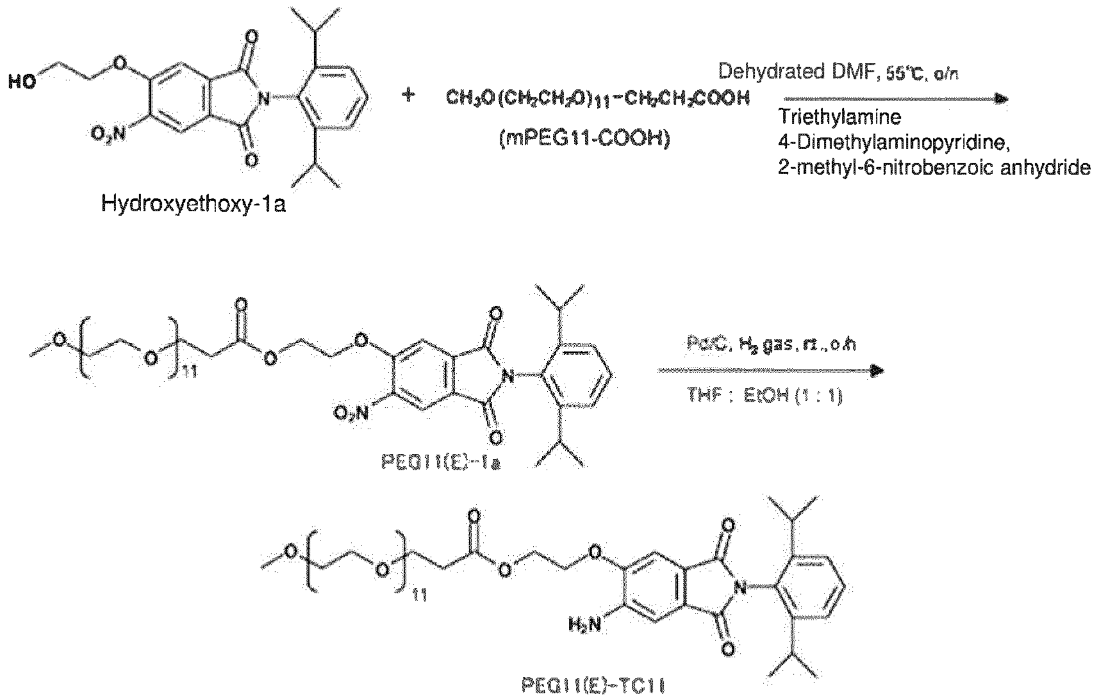


Hydroxyethoxy-1a

PEG11(E)-1a

PEG11(E)-TC11

The compound of the present invention, PEG11(E)-TC11 prepared via modification with PEG, can be obtained as follows. For example, 4-hydroxyphthalic acid is nitrated with guanidine nitrate to give a mixture of a 5-nitro compound and a 3-nitro compound of 4-hydroxyphthalic acid, and then the mixture is dehydrated with phosphorus pentoxide so as to be able to give a hydroxyphthalic anhydride mixture of a 5-nitro anhydride and a 3-nitro anhydride. The mixture is reacted with 2,6-diisopropylaniline, so as to be able to give a diisopropylphenyl phthalimide derivative (1a and 1b). The mixture is purified by silica gel chromatography, so that 1a and 1b can be separated from each other. 1a is reacted with ethylene bromohydrin, so as to be able to give hydroxyethoxy-la. The hydroxyethoxy-la is esterified with mPEG11-COOH to give PEG11(E)-1a, and then PEG11(E)-1a is subjected to catalytic reduction with a hydrogen gas in the presence of a Pd/C catalyst, so that PEG11(E)-TC11 can be finally obtained.

PEG11(0)-TC11 of the present invention can be produced by a method for producing the TC11 derivative (PEG11(O)-TC11) through PEGylation ofTC11 via an ether bond such as the following method when a reactive group for modification is mPEG11.

[Chemical formula 5]

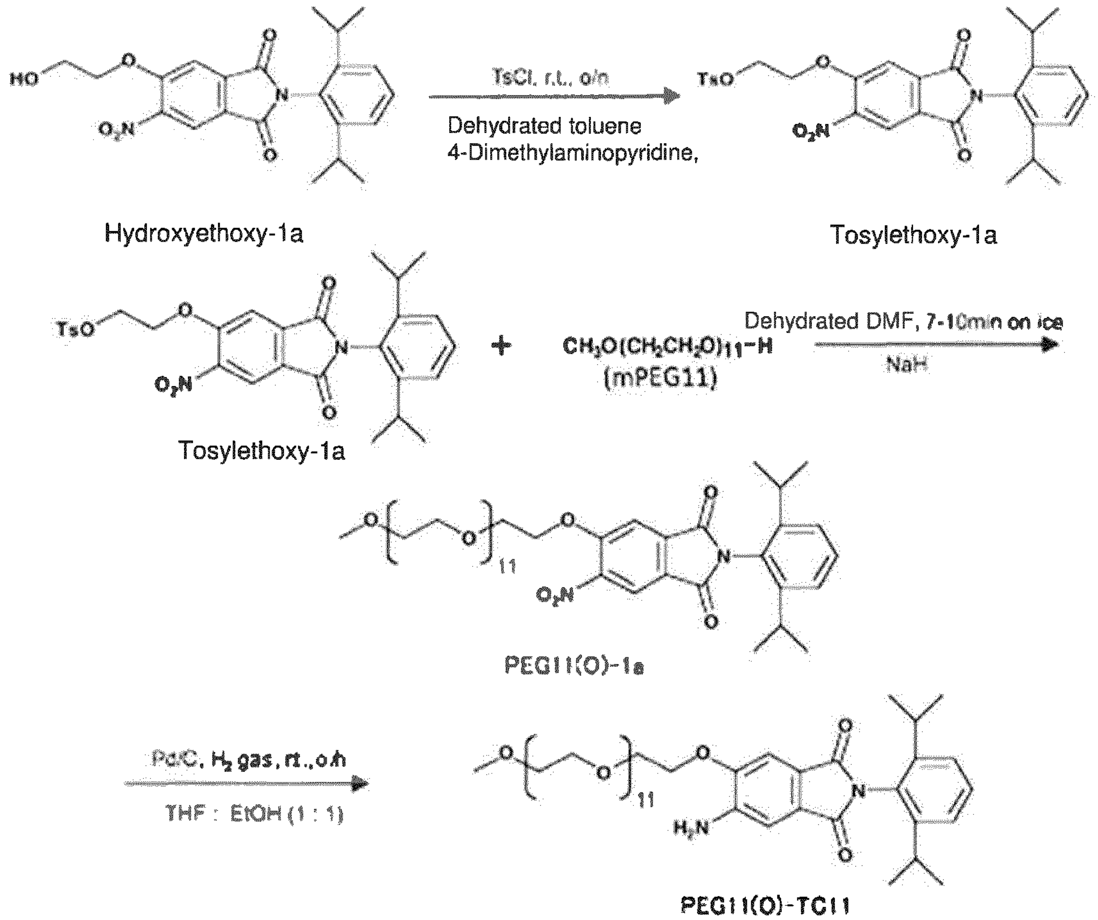


Hydroxyethoxy-1a

Tosylethoxy-1a

Tosylethoxy-1a

PEG11(O)-1a

PEG11(O)-TC11

Hydroxyethoxy-1a synthesized in advance is tosylated with para-toluenesulfonyl chloride (TsCl) to synthesize tosylethoxy-1a, mPEG11 is reacted with the resultant to obtain PEG(O)-1a, and then catalytic reduction is finally performed with a hydrogen gas in the presence of a Pd/C catalyst, so that PEG11(O)-TC11 can be obtained.

A derivative having another substituent, such as PEG5(E)-TC11 can be similarly produced by adequately substituting a starting material or the like.

<2> Pharmaceutical composition of the present invention

The present invention provides a pharmaceutical composition comprising the above derivative. The pharmaceutical composition is preferably an anticancer agent. The anticancer agent is preferably used for multiple myeloma.

A phenylphthalimide derivative as an active ingredient can be formulated into a preparation (pharmaceutical composition) using a pharmaceutically acceptable carrier. Examples of such a pharmaceutically acceptable carrier include an excipient and a base. Moreover, the preparation may contain a generally employed additive. The dosage form thereof is selected as appropriate depending on the route of administration. For example, the phenylphthalimide derivative may be formulated into preparations such as tablets, capsules, granules, powders, or syrups for peroral administration, or can be formulated into preparations such as injection preparations, or suppositorries for parenteral administration via intraperitoneal, intravenous, or subcutaneous injection. Examples of the preparation also include a preparation prepared by packaging the phenylphthalimide derivative as an active ingredient and another anticancer agent separately and then integrating the packages into a single preparation. The anticancer agent of the present invention can be administered to a patient who is receiving or will receive anticancer drug treatment. Such a phenylphthalimide derivative may be used independently or in combination with another drug (for example, another anticancer agent).

The dosage of the phenylphthalimide derivative is selected as appropriate depending on target anticancer drug treatment, patient's conditions, and the like. For example, when the phenylphthalimide derivative is administered to a

human, the phenylphthalimide derivative can be administered at a dosage of about 0.1 to 20 mg/kg (body weight) per day, preferably about 0.1 to 0.5 mg/kg (body weight) per day in a single dose or several doses via peroral administration or parenteral administration including intravenous administration and subcutaneous administration. The dosage and the frequency of administration can be varied adequately depending on the symptom, age, and the method of administration, for example.

The phenylphthalimide derivative can be used for treatment of cancers including, particularly, hematologic malignancies such as myeloma, malignant lymphoma, leukemia, and myelodysplastic syndrome and solid cancers such as colon cancer, lung cancer, renal cell cancer, mammary cancer, brain tumor, ovarian carcinoma, melanoma, stomach cancer, and prostate cancer. In particular, the phenylphthalimide derivative can be advantageously used for multiple myeloma.

EXAMPLES

The present invention will be described in detail as follows with reference to the following examples. These examples are preferred embodiments of the present invention, and the present invention is not limited to these examples.

Example 1

Synthesis of PEG11(E)-TC11

Step 1

Nitration of 4-hydroxyphthalic acid

4-Hydroxyphthalic acid (2.5 g, 13.73 mmol) was added to a 300-ml three-necked flask, and then the mass was pulverized using a glass rod into powders as fine as possible. 88 ml of 85% sulfuric acid (96% sulfuric acid was added to 10 ml of $H_2O$ to give 88 ml of the resultant.) was added and then the resulting mass was subjected to ultrasonication, stirring, or pulverization with a glass rod, followed by complete dissolution at room temperature. Guanidine nitrate (1.68 g, 13.76 mmol) was gradually added while stirring on ice, and then the resultant was further stirred for 3 hours while cooling with ice. The reaction solution was poured into ice-cold $H_2O$ (400 ml), and then the flask was washed with 80 ml of ice-cold $H_2O$. The total amount was about 550 ml. The solution was poured into a 1-L separatory funnel, and then extracted twice with ethyl acetate (170 ml × 2). The resulting ethyl acetate layer was washed twice with a saturated saline solution (70 ml × 2), dehydrated with anhydrous sodium sulfate, and then filtered. The filtrate was evaporated to dryness.

[Chemical formula 6]

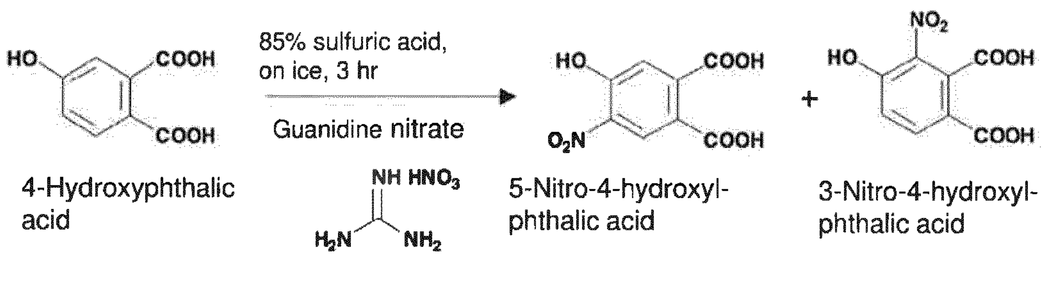


4-Hydroxyphthalic acid

5-Nitro-4-hydroxyl-phthalic acid

3-Nitro-4-hydroxyl-phthalic acid

The nitrated product 3.66 g (13.15 mmol, yield 95.8%) was obtained as a pale yellow powder by the above step. As a result of $^1$H-NMR spectroscopy in deuterated methanol, the reaction product was found to be an about 1:1 mixture of 5-nitro-4-hydroxyphthalic acid and 3-nitro-4-hydroxyphthalic acid.

The Rf values of silica gel TLC (developing solvent: chloroform/methanol = 1:1): The Rf value of the raw material (4-hydroxyphthalic acid) was 0.77, the same of the product, 5-nitro compound, was 0.3, and the same of 3-nitro compound was 0.07.

$^1$H NMR (400 MHz, $CDCl_3$): 5-nitro compound: δ8.56 (s, 1H), 7.27 (s, 1H); 3-nitro compound: δ8.00 (d, J =8.8 Hz, 1H), 7.14 (d, J =8.8 Hz, 1H).

The product ratio of the 5-nitro compound and the 3-nitro compound was 1:1 as a result of $^1$H NMR spectroscopy.

Step 2

Synthesis of 5-nitro and 3-nitro-4-hydroxyphthalic anhydrides

A mixture (1.22 g, 5.37 mmol) of 5-nitro-4-hydroxyphthalic acid and 3-nitro-4-hydroxyphthalic acid were added to a 500-ml three-necked flask, and then the mass was pulverized using a glass rod. After 1 hour of drying under vacuum, dehydrated toluene (150 ml) was added. However, the resultant was practically insoluble and thus was subjected to ultrasonication to give a suspension that was homogenous as possible. SICAPENT (14.4 g, as $P_2O_5$: 10.8 g, 76.08 mmol) was weighed in a beaker, and then immediately added into the flask under a nitrogen gas using a funnel and a spatula. Since SICAPENT is highly hygroscopic, it should be added quickly. The funnel was washed with the remaining dehydrated toluene (33 ml), and then under an argon gas, heated under reflux at 100°C for a whole day and night. After cooling to room temperature, the reaction solution was filtered off, the resultant was washed with toluene, and then the filtrate (with slight clouding) was evaporated to dryness.

[Chemical formula 7]

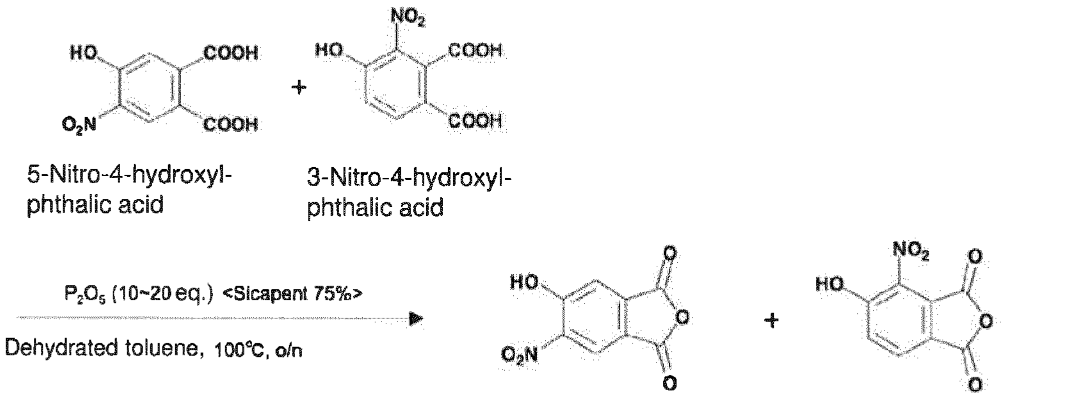


5-Nitro-4-hydroxyl-phthalic acid + 3-Nitro-4-hydroxyl-phthalic acid

5-Nitro-4-hydroxyphthalic anhydride + 3-Nitro-4-hydroxyphthalic anhydride

The reaction product 571.6 mg (2.73 mmol, yield 50.8%) was obtained as a pale yellow powder by the above step. The Rf values of silica gel TLC (developing solvent: chloroform/methanol = 1:1): The Rf value of the raw material (5-nitro-4-hydroxyphthalic acid) was 0.27, the same of (3-nitro-4-hydroxyphthalic acid) was 0.1, the same of 5-nitro-4-hydroxyphthalic anhydride was 0.9, and the same of 3-nitro-4-hydroxyphthalic anhydride was 0.77.
$^1H$ NMR (400 MHz, $CDCl_3$): 5-nitro compound: δ8.82 (s, 1H), 7.81 (s, 1H); 3-nitro compound: δ8.14 (d, J =8.8 Hz, 1H), 7.70 (d, J =8.8 Hz, 1H).

The product ratio of the 5-nitro compound and the 3-nitro compound was 1:0.6 as a result of $^1H$ NMR spectroscopy.

Step 3

Addition of 2,6-diisopropylaniline

2,6-Diisopropylaniline (587mg, 3.31mmol) was weighed in a 50-ml two-necked eggplant flask, acetic acid (2 ml) was added, and then the mixture was stirred. A mixture (571.6 mg, 2.73 mmol) of 5-nitro and 3-nitro-4-hydroxyphthalic anhydrides was added to and dissolved in the remaining acetic acid (11.5 ml), followed by heating for a whole day and night under reflux at 140°C. After cooling to room temperature, the reaction solution was poured into 75 ml of $H_2O$ (in an amount about 5 times that of acetic acid), to immediately give a beige fine precipitate. The solution was gently stirred for 30 minutes, and then the resulting precipitate was filtered off (see note). After washing with 100 ml of 5% acetic acid, the filter paper including the precipitate was dried.

[Chemical formula 8]

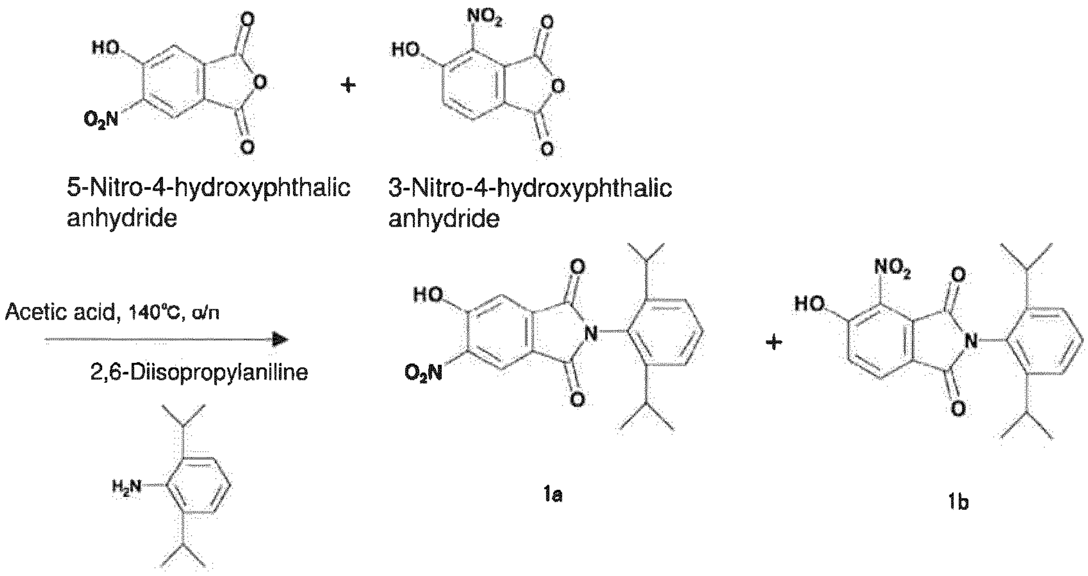


5-Nitro-4-hydroxyphthalic anhydride

3-Nitro-4-hydroxyphthalic anhydride

1a

1b

A light brown powder (687 mg) was obtained by the above step. The powder adhered to the filter paper was dissolved with ethyl acetate and then evaporated to dryness, so that 273 mg of a similar light brown powder was obtained. A total of 0.96 g of the product (2.61 mmol, yield 95.6%) was obtained. As a result of $^1$H-NMR spectroscopy in deuterated chloroform, the resultant was a mixture (compound 1a and compound 1b) in which 2,6-diisopropylaniline was added to the 5-nitro compound and to the 3-nitro compound, respectively.

The Rf values of silica gel TLC (developing solvent: chloroform/methanol=10:1): The Rf value of the raw material (5-nitro-4-hydroxyphthalic anhydride) was 0.14, the same of (3-nitro-4-hydroxyphthalic anhydride) was 0.07, the same of the product, 1a, was 0.34, and the same of 1b was 0.17.

(Note) The precipitate that appears herein is so fine to easily cause severe clogging upon filtration. In such a case, extraction with ethyl acetate was performed. The ethyl acetate layer contained 1a and 1b, unreacted aniline, and acetic acid. Hence, the layer was evaporated to dryness so as to remove acetic acid as much as possible. Aniline could be removed in the next process, silica gel chromatography.

The mixture of 1a and 1b (0.96 g) was dissolved in 1.8 ml of chloroform, sprinkled with silica gel, and then injected into the upper part of a silica gel column (φ3.5 cm × 10.5 cm; 100.8 ml) subjected to swelling in hexane:ethyl acetate (40:1). Aniline mixed therein was eluted with hexane:ethyl acetate (10:1), and then the resultant was eluted with hexane:ethyl acetate (4:1).

[Chemical formula 9]

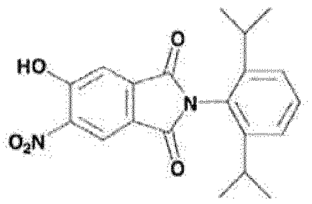
+
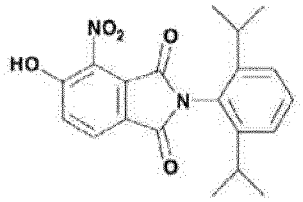

1a 1b

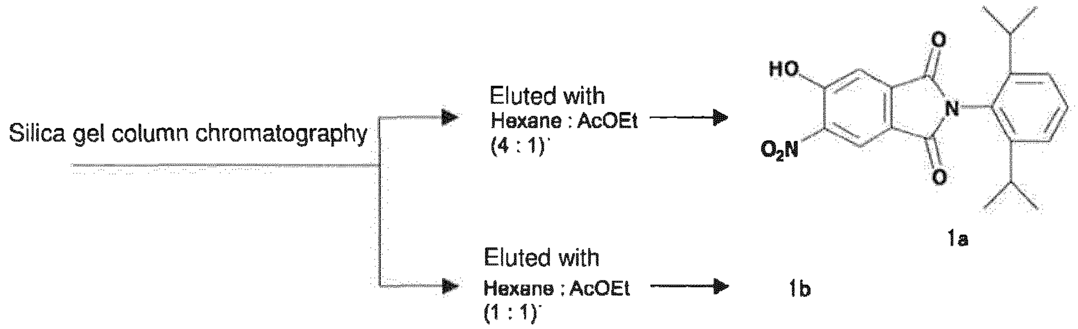


A yellow substance with Rf=0.34 was obtained by silica gel TLC (chloroform/methanol=10:1) in the above step. The purified product was identified as 1a by $^1$H-NMR spectroscopy in deuterated chloroform. Moreover, under the above TLC conditions, only a single spot of 1a was confirmed. The yield was 628.8 mg (31.8%).

$^1$H NMR (400 MHz, $CDCl_3$): 5-nitro compound :δ8.76 (s, 1H), 7.71 (s, 1H), 7.48 (t, 7.6 Hz, 1H), 7.29 (d, J =7.6 Hz, 2H), 2.61 (sep, J =7.2 Hz, 2H), 1.16 (d, J =7.2 Hz, 12H); 3-nitro compound :δ8.11 (d, J =8.8 Hz, 1H), 7.59 (d, J =8.8 Hz,1H), 7.48 (t, J =7.6 Hz, 1H), 7.30 (d, J =7.6 Hz, 2H), 2.63 (sep, J =7.2 Hz, 2H), 1.16 (d, J =7.2 Hz, 12H) The product ratio of the 5-nitro compound and the 3-nitro compound was 1:0.7 as a result of $^1$H NMR spectroscopy.
MS(EI):$[M]^+$ m/z 368

Step 4

Addition of hydroxyethoxy group to 1a

1a of the 5-nitro compound (628.8 mg, 1.71 mmol) was weighed in a 100-ml two-necked eggplant flask, dried under vacuum for 1 to 2 hours, and then dissolved in dehydrated DMF (24 ml). Diisopropylethylamine (583.4 μl, 3.40 mmol) and ethylene bromohydrin (240.5 μl, 3.41 mmol) were added, and then the mixture was heated at 110°C for a whole day and night under reflux. After the temperature returned to room temperature, the resultant was transferred to a 300-ml separatory funnel. 2 M HCl (60 ml) was added and then extraction was performed with hexane:ethyl acetate (1:1) (60 ml×3). The results of silica gel TLC confirmed the reaction product in the hexane:ethyl acetate layer. The hexane:ethyl acetate layer was washed with a saturated saline solution. After dehydration with anhydrous sodium sulfate, the resultant was filtered and then subjected to vacuum concentration, so that 701 mg of a residue (dark brown viscous liquid) was obtained. The thus obtained residue contained a reaction product and unreacted raw materials.

The thus obtained residue was dissolved in 0.9 ml of chloroform, sprinkled with silica gel, and then injected into the upper part of a silica gel column (φ3.5 cm×7.2 cm; 69.1 ml) subjected to swelling in hexane:ethyl acetate (40:1). The column was washed sequentially with hexane:ethyl acetate (40:1) and then hexane:ethyl acetate (10:1), and then unreacted 1a was eluted with hexane:ethyl acetate (4:1). Subsequently, the target reaction product was eluted with hexane:ethyl acetate (3:1).

[Chemical formula 10]

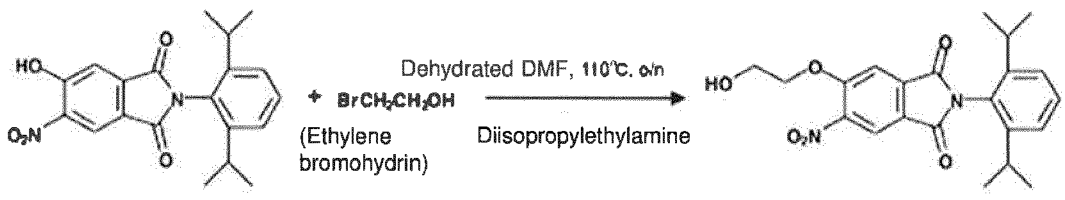


1a Hydroxyethoxy-1a

The reaction product was identified as hydroxyethoxy-la as a result of $^1$H-NMR spectroscopy in deuterated chloroform. The yield of hydroxyethoxy-1a was 567.2 mg (1.38 mmol, 80.7%).

The Rf values of silica gel TLC (developing solvent: chloroform/ethyl acetate = 1:1): The Rf value of hydroxyethoxy-1a was 0.67, and the same of raw material 1a was 0.1.

$^1$H NMR (400 MHz, $CDCl_3$): δ8.37 (s, 1H), 7.71 (s, 1H), 7.48 (t, J =7.8 Hz, 1H), 7.30 (d, J =7.8 Hz, 2H), 4.43 (t, J =4.3 Hz, 2H), 4.07 (m, 2H), 2.65 (sep, J =6.8 Hz, 2H), 1.17 (d, J =6.8 Hz, 12H).

MS(EI):$[M]^+$ m/z 412

Step 5

Esterification of hydroxyethoxy-la and mPEG11-COOH

Hydroxyethoxy-la (228.4 mg, 0.554 mmol) was weighed in a 20-ml pear-shaped flask and mPEG11-COOH (401.5 mg, 0.682 mmol) was weighed in a 10 ml pear-shaped flask, followed by about 1 hour of drying under vacuum. Dehydrated DMF (1.6 ml) and triethylamine (257 μl, 1.85 mmol) were added to a 50-ml two-necked eggplant flask, and then 4-dimethylaminopyridine (6.96 mg, 0.057 mmol) and 2-methyl-6-nitrobenzoic anhydride (235.1 mg, 0.683 mmol) were further added under a nitrogen gas flow. Previously dried mPEG11-COOH was added and dissolved in 2.8 ml of dehydrated DMF, followed by 15 minutes of stirring at room temperature. Subsequently, previously dried hydroxyethoxy-la was added to and dissolved in 5.5 ml of dehydrated DMF, and then the resultant was heated at 55°C for a whole day and night under reflux. After reaction, the resultant was transferred to a 50-ml eggplant flask, and then DMF was evaporated to dryness at 55°C and 10 mmHg. The residue (brown viscous liquid) was dissolved in ethyl acetate (40 ml), the solution was transferred to a 200-ml separatory funnel, and then saturated ammonium chloride (pH 4) (40 ml) was added for extraction. Extraction was further performed twice with ethyl acetate (40 ml), the ethyl acetate layer was neutralized with 50 ml of saturated sodium hydrogen carbonate (pH 8), washed with a saturated saline solution, dehydrated with anhydrous sodium sulfate, filtered, and then evaporated to dryness.

[Chemical formula 11]

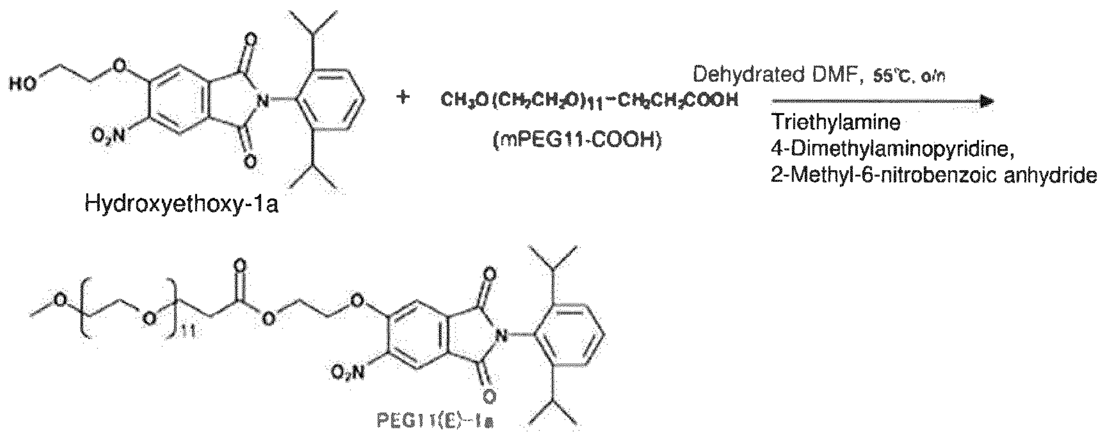


Hydroxyethoxy-1a

PEG11(E)-1a

Finally, 546.8 mg of a residue was obtained. The thus obtained residue was dissolved in 0.5 ml of chloroform, and then injected into the upper part of a silica gel column (φ2.5 cm × 10 cm; 49 ml) subjected to swelling in chloroform. Unreacted hydroxyethoxy-la was eluted with chloroform (100%), and then a target reaction product was eluted with chloroform:methanol (98:2). The results of $^1$H-NMR spectroscopy in deuterated chloroform confirmed that the product was PEG11(E)-1a. The result of silica gel TLC (chloroform/methanol=10:1) confirmed the spot of only PEG11(E)-1a.

The yield was 365 mg (0.371 mmol, 67%).

The Rf values of silica gel TLC (developing solvent: chloroform/methanol=10:1): The Rf value of the raw material, hydroxyethoxy-la, was 0.53, and the same of the product, PEG1 1(E)-1a, was 0.47.

$^1H$ NMR (400 MHz, $CDCl_3$): δ8.37 (s, 1H), 7.71 (s, 1H), 7.48 (t, J =7.6 Hz, 1H), 7.30 (d, J =7.6 Hz, 2H), 4.53 (m, 4H), 4.07 (m, 2H), 3.77 (t, J =6.4 Hz, 2H), 3.65 (br.s, 44H), 3.55 (dd, J =2.8, 4.0 Hz, 2H), 3.38 (s, 3H), 2.65 (sep, J =6.4 Hz, 2H), 1.16 (d, J =6.4 Hz,12H).

Step 6

Synthesis of PEG11(E)-TC11 by hydrogenation reaction of PEG11(E)-1a

PEG11(E)-1a (365 mg, 0.371 mmol) was added to a 200-ml two-necked eggplant flask, and then dissolved in 43 ml of tetrahydrofuran:ethanol (1:1). After addition of 5% Pd/C (172.5 mg) under an argon gas flow, substitution with a hydrogen gas was performed, and then to stirring at room temperature for a whole day and night. The reaction solution was filtered using sellite, and then the filtrate was evaporated to dryness, so that 402.1 mg of a residue (yellow green viscous liquid) was obtained. The residue was dissolved in chloroform (0.6 ml), and then the resultant was injected into the upper part of a silica gel column (φ2.5 cm×9.5 cm; 46.6 ml) subjected to swelling in chloroform. After the column was washed with 100% chloroform, and then with chloroform:methanol (99:1), a target reaction product was eluted with chloroform:methanol (98:2), and thus a fluorescent pale yellow oily substance was obtained.

[Chemical formula 12]

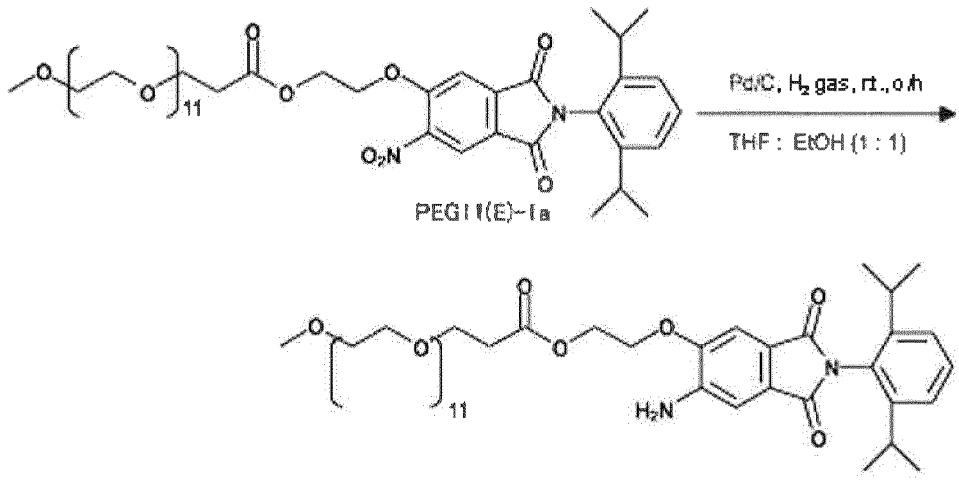


PEG11(E)-1a

PEG11(E)-TC11

The thus obtained substance was identified as PEG11(E)-TC11 as a result of $^1H$-NMR spectroscopy in deuterated chloroform. The results of silica gel TLC confirmed a single spot of only PEG11(E)-TC11. The yield was 386.8 mg (0.406 mmol), and the yield (%) was 73.3% relative to the starting material, hydroxyethoxy-1a.

The Rf values of silica gel TLC (developing solvent: chloroform/methanol=10:1) (Note): The Rf value of the raw material, PEG(E)-1a, was 0.47, and the same of the product, PEG11(E)-TC11, was 0.47.

(Note) The two exhibited almost the same Rf value as a result of silica gel TLC under the conditions. PEG11(E)-TC11 emitted yellow fluorescence by irradiation at 365 nm, but PEG11(E)-1a emitted no fluorescence. Moreover, $^1H$-NMR results revealed that the raw materials were all hydrogenated to give PEG11(E)-TC11.

$^1H$ NMR (400 MHz, $CDCl_3$): δ7.25 (s, 1H), 7.16 (s, 1H), 7.42 (t, J =7.8 Hz, 1H), 7.26 (d, J =7.8 Hz, 2H), 4.56 (t, J =3.6 Hz, 2H), 4.33 (t, J =3.6 Hz, 2H), 3.78 (t, J =6.4 Hz, 2H), 3.64 (br.s, 44H), 3.55 (dd, J = 2.8, 6.0 Hz, 2H), 3.38 (s, 3H), 2.66 (sep, J =6.8 Hz, 2H), 1.14 (d, J =6.8 Hz,12H).

HRMS(ESI): The calculated value of m/z $[M+Na]^+$ was 975.50417, and the measured value was 975.50454.

HPLC: Analysis with a YMC-Pack C18 column (250 mm × 4.6 mm, particle diameter of 5 μm, flow rate of 0.7 ml/min, solvent; $CH_3CN$: $H_2O$=1:1) revealed elution at 11.8 min.

UVmax = 270(s), 317(w), 404(w) nm (50% EtOH)

Example 2

Synthesis of PEG5(E)-TC11

PEG5(E)-TC11 was synthesized from the hydroxyethoxy-la by the following method.

Step 1

Esterification of hydroxyethoxy-1a and mPEG5-COOH

[Chemical formula 13]

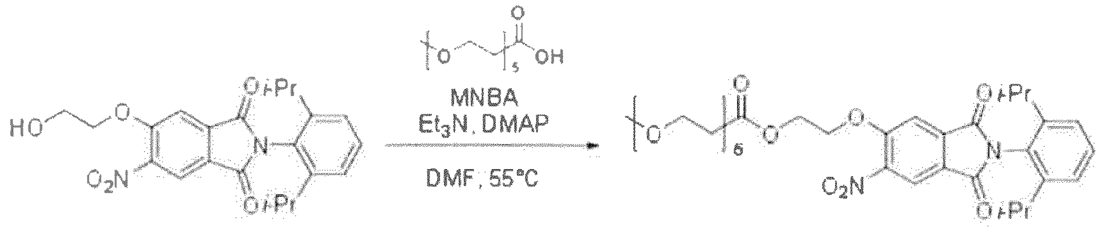


Hydroxyethoxy-1a
Mol Wt. 412.44

PEG5(E)-1a
Mol Wt: 674.74

DMAP 50.0 mg (0.436 mmol) and 2.0 mL (14.53 mmol) of triethylamine were dissolved in 20 mL of DMF, and then 1.8 g (4.36 mmol) of hydroxyethoxy-la and 1.5 g (5.37 mmol) of mPEG5-COOH were added. Subsequently, 1.85 g (5.37 mmol) of MNBA was added, the reaction solution was heated to 55°C under an argon atmosphere, and then stirred for 16 hours. Completion of the reaction was confirmed by HPLC, water was added, extraction with ethyl acetate was performed, and then an aqueous layer was further extracted with ethyl acetate. An organic layer was washed with a saturated saline solution, dried using sodium sulfate, and then subjected to vacuum concentration.

The resultant was purified by silica gel chromatography (HiFlash 2L heptane/ethyl acetate = 5/1→0/1), and then the main distillate was subjected to vacuum concentration, so that 2.86 g of PEG5(E)-1a was obtained as a colorless oil (yield: 97.3%).

Step 2

Synthesis of PEG5(E)-TC11 by hydrogenation reaction of PEG5(E)-1a

[Chemical formula 14]

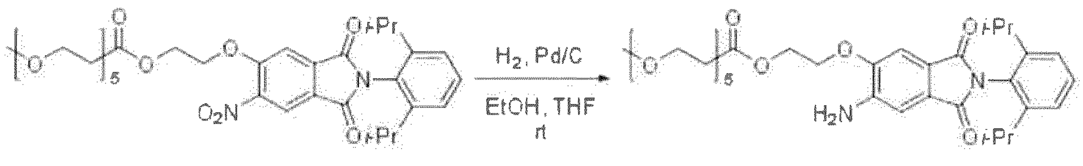


PEG5(E)-1a
Mol Wt: 674.74

PEG5(E)-TC11
Mol Wt: 644.76

After addition of 450 mg of Pd/C (K-type) to a solution (mixture of 56 mL of EtOH and 56 mL of THF) of 2.86 g (4.23 mmol) of PEG5(E)-1a, the resultant was stirred for 2 hours under a hydrogen atmosphere at room temperature.

After the completion of the reaction was confirmed by HPLC, the reaction solution was filtered and then concentrated under vacuum. The resultant was purified by silica gel chromatography (HiFlash 3 L methanol/chloroform = 0/1→1/20) and then the main distillate was concentrated under vacuum, thereby obtaining 2.54 g of PEG5(E)-TC11 as a yellow oil (yield: 93.0%).

HPLC: Analysis with an YMC-Pack Pro C18 column (250 mm × 4.6 mm, particle diameter of 5 μm, flow rate of 1.0 ml/min, solvent; A: $H_2O$ containing 0.1% TFA, B: AcCN containing 0.1% TFA, Gradient: A/B= 100/30→50/50 (10 min)→15/75 (15 min)→0/100 (20 min)→100/30 (25 min), revealed elution at 20.5 min.
MS (TOFMS): $[M]^+$m/z 644.6
$^1$H-NMR(400 MHz, d6-DMSO): δ7.44(t, J=7.6 Hz, 1H),7.35(s, 1H), 7.30(d, J=7.6 Hz, 2H), 7.09(s, 1H), 6.11(br.s, 2H), 4.44(br.t, 2H), 4.39(br.t, 2H), 3.65(t,J=6.4Hz), 3.48(m, 16H),3.23(s,3H),2.64(sep, J=7.2 Hz,2H),1.06(d, J=8.4 Hz,12H) .

Example 3

Synthesis of PEG11(O)-TC11

Step 1

Tosylation of hydroxyethoxy-1a

Hydroxyethoxy-1a (666.6 mg, 1.62 mmol) was weighed in a 100-ml eggplant flask, dried under vacuum for about 1 hour, and then dissolved by the addition of dehydrated toluene (43 ml). While stirring on ice, para-toluene sulfonyl chloride (623.3 mg, 3.27 mmol) was added under a nitrogen gas flow. 4-dimethylaminopyridine (400.2 mg, 3.28 mmol) was subsequently added to cause precipitation, but left to stand until the temperature returned to room temperature, followed by stirring for a whole day and night. After reaction, the precipitate was filtered off using sellite, and then the filtrate was evaporated to dryness, thereby obtaining 818.6 mg of a residue. The results of silica gel TLC (hexane:ethyl acetate =1:1) confirmed the spot of tosylethoxy-1a (Rf=0.75), few spots of para-toluenesulfonyl chloride (Rf=0.82) and the spot of a small amount of hydroxyethoxy-1a(Rf=0.49).

[Chemical formula 15]

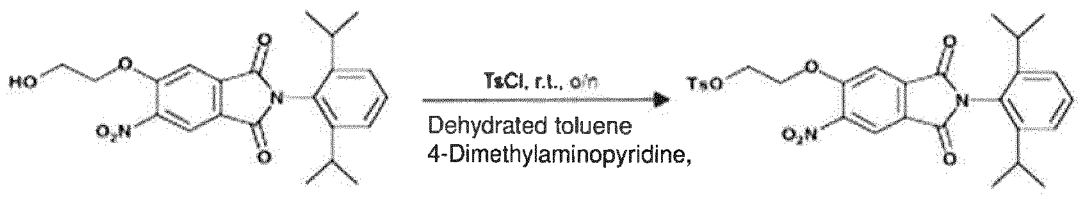


Hydroxyethoxy-1a  Tosylethoxy-1a

The thus obtained residue was dissolved in 1.5 ml of chloroform, sprinkled with silica gel, and then injected into the upper part of a silica gel column (ϕ)3.5 cm × 6.7 cm; 64.3 ml) subjected to swelling in hexane. After elution of para-toluenesulfonyl chloride with hexane:ethyl acetate (5:1), a target reaction product was eluted with hexane:ethyl acetate (4:1). Unreacted hydroxyethoxy-la was collected with hexane:ethyl acetate (2:1). The product was identified as tosylethoxy-1a by $^1$H-NMR spectroscopy in deuterated chloroform. The yield was 503.7 mg (0.888 mmol, 55%) and the same of the unreacted hydroxyethoxy-la was 144.4 mg (0.35 mmol, 22%).

The Rf values of silica gel TLC (developing solvent: hexane/ethyl acetate =1:1): The Rf value of the raw material, hydroxyethoxy-la, was 0.49, the same of the product, tosylethoxy-1a, was 0.75, and the same of para-toluenesulfonyl chloride was 0.82.
$^1$H NMR (400 MHz, $CDCl_3$): δ8.30 (s, 1H), 7.82 (d, J =6.5 Hz, 2H), 7.60 (s, 1H), 7.48 (t, J =6.4 Hz, 1H), 7.39 (d, J =6.5 Hz, 2H), 7.30 (d, J =6.4 Hz, 2H), 4.50 (m, 2H), 4.45 (m, 2H), 2.63 (sep, J =5.2 Hz, 2H), 1.16 (d, J =5.2 Hz, 12H).

Step 2

Etherification of tosylethoxy-1a and mPEG11

Molecular sieve 3A (1.0 g) was weighed in a small beaker. Twenty five seconds of microwave heating and 20 minutes of cooling under vacuum were repeated 3 times to activate molecular sieve 3A, and then the resultant was added to a 20-ml two-necked eggplant flask under a nitrogen gas flow. mPEG11 (307.6 mg, 0.595 mmol) was weighed in a 20 ml pear-shaped flask, decompressed for 1 hour, dissolved in dehydrated DMF (2 ml), added to the flask of molecular sieve 3A, and then stirred at room temperature for 30 minutes to 1 hour. Sodium hydride (46.4 mg, 27.8 mg

as sodium hydride, 1.16 mmol) was weighed in a 10-ml two-necked eggplant flask. Mineral oil adhered thereto was washed 3 times with dehydrated hexane, dried under vacuum, and then suspended in dehydrated DMF (2.9 ml). The previous DMF solution of mPEG11 was stirred on ice for cooling. The suspension of sodium hydride was slowly added dropwise to the solution and then the solution was stirred at room temperature for 1.5 hours. Subsequently, tosylethoxy-1a (157.9 mg, 0.278 mmol) weighed in advance in a 20 ml pear-shaped flask with stirring on ice, dried under vacuum for 2 to 3 hours and then dissolved in dehydrated DMF (1.4 ml) was added dropwise. The resultant was stirred on ice for 7 to 10 minutes. Next, a supernatant of the reaction solution was transferred into a separatory funnel, to which 0.1 M phosphate buffer pH7.0 (5 ml) had been added. The reaction flask and the molecular sieve were washed with ether (12 ml), and then the first extraction was performed. Furthermore, a small amount of a saturated saline solution was added to the aqueous layer, and then extraction was performed twice with ether (12 ml). The ether layer was washed 3 times with a saturated saline solution, dehydrated with anhydrous sodium sulfate, and then filtered, followed by evaporation to dryness.

[Chemical formula 16]

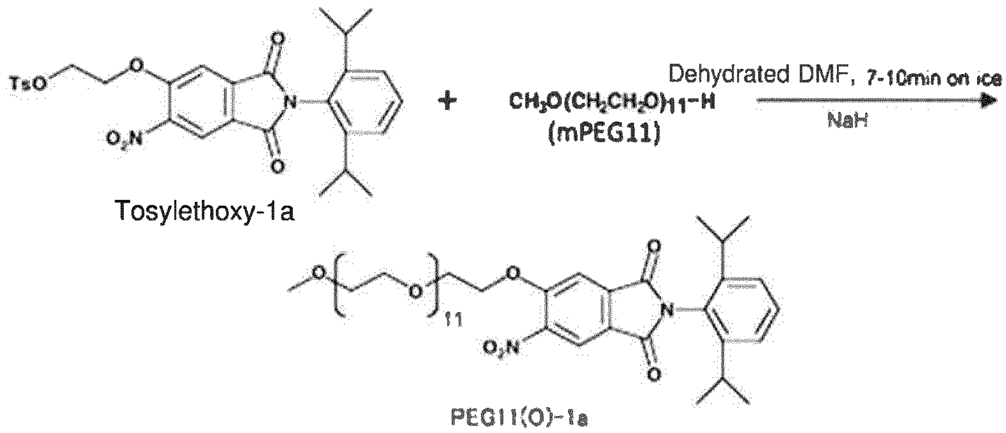


Tosylethoxy-1a

PEG11(O)-1a

A residue (193.9 mg) (orange viscous liquid) was obtained by the above step. The results of silica gel TLC (chloroform:methanol=10:1) confirmed the spot of the target PEG11(O)-1a having UV at Rf=0.42, and the spot having weak UV at Rf=0.94.

The residue was dissolved in chloroform (0.8 ml), and then injected into the upper part of a silica gel column (φ1.3 cm × 11 cm; 14.6 ml) subjected to swelling in chloroform. The column was sufficiently washed with chloroform, and then a target product was eluted with chloroform:methanol (98:2). As a result of $^1$H-NMR spectroscopy in deuterated chloroform, the product was identified as PEG11(O)-1a. The results of silica gel TLC confirmed the spot of only PEG11(O)-1a (UV absorption and at the lower side thereof, extremely weak pale fluorescence was present). The yield was 165.5 mg (0.182 mmol, 65.5%).

The Rf values of silica gel TLC (developing solvent: chloroform/methanol = 10:1): The Rf value of the raw material, tosylethoxy-1a, was 0.94, and the same of the product, PEG11(O)-1a, was 0.42.
$^1$H NMR (400 MHz, $CDCl_3$): δ8.30 (s, 1H), 7.73 (s, 1H), 7.47 (t, J =6.2 Hz, 1H), 7.30 (d, J =6.2 Hz, 2H), 4.45 (t, J =3.7 Hz, 2H), 3.96 (t, J =3.7 Hz,2H), 3.64 (br.s, 44H), 3.34 (s, 3H), 2.65 (sep, J =5.6 Hz, 2H), 1.16 (d, J =5.6 Hz,12H).

Step 3

Synthesis of PEG11(O)-TC11 by hydrogenation reaction of PEG11(O)-1a

PEG11(O)-1a (165.5 mg, 0.182 mmol) was added to a 200-ml two-necked eggplant flask, and then dissolved in 40 ml of tetrahydrofuran:ethanol (1:1). After addition of 5% Pd/C (135.6 mg) under an argon gas flow, substitution with a hydrogen gas was performed, and then the resultant was stirred at room temperature for a whole day and night. The reaction solution was filtered using sellite, and then the filtrate was evaporated to dryness.

[Chemical formula 17]

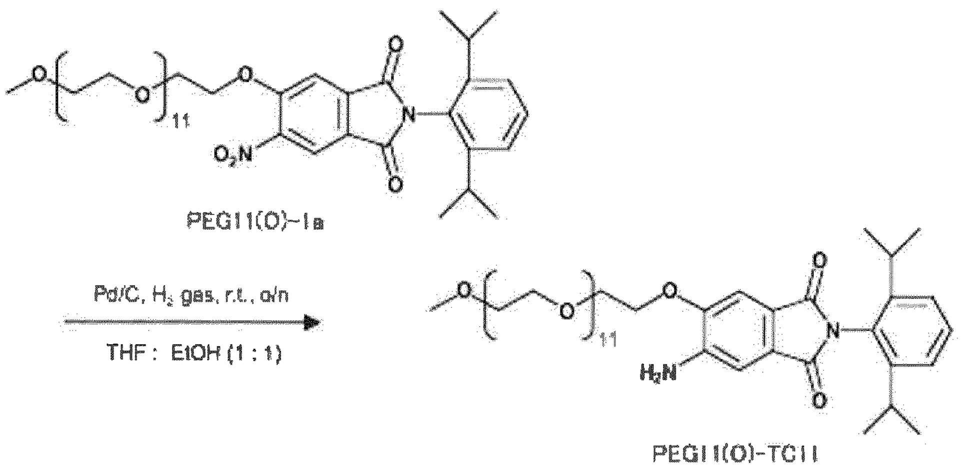


PEG11(O)-1a

PEG11(O)-TC11

A residue (154.6 mg) (yellow green viscous liquid) was obtained by the above step. The results of silica gel TLC (chloroform:methanol = 10:1) confirmed the spot (yellow fluorescence) of the target product, PEG11(O)-TC11, at Rf=0.39. Weak pale fluorescence was observed on the upper and the lower sides of the spot.

The residue was dissolved in chloroform (0.7 ml), and then injected into the upper part of a silica gel column (φ1.3 cm × 10 cm; 13.3 ml) subjected to swelling in chloroform. The column was washed with 100% chloroform and then with chloroform:methanol (99:1) (green and pale fluorescent substances were removed), and then a target reaction product was eluted with chloroform:methanol (98:2), thereby obtaining a fluorescent yellow oily substance.

The results of silica gel TLC confirmed only a single spot (Rf=0.39) of PEG11(0)-TC11 turned yellow having orange UV and yellow fluorescence. As a result of $^1$H-NMR spectroscopy in deuterated chloroform, the substance was identified as PEG11(O)-TC11. The yield was 145.9 mg (0.166 mmol), and the yield (%) was 32.9% relative to the starting material, hydroxyethoxy-la.

The Rf values of silica gel TLC (developing solvent: chloroform/methanol=10:1): The Rf value of the raw material, PEG11(O)-1a, was 0.42, and the same of the product, PEG11(0)-TC11 was 0.39.

$^1$H NMR (400 MHz, $CDCl_3$): δ8.11 (s, 1H), 7.53 (s, 1H), 7.44 (t, J =6.2 Hz, 1H), 7.27 (d, J =6.2 Hz, 2H), 4.30 (t, J =4.6 Hz, 2H), 3.96 (t, J =4.6 Hz,2H), 3.64 (br.s, 44H), 3.35 (s, 3H), 2.66 (sep, J =5.6 Hz, 2H), 1.14 (d, J =5.6 Hz,12H).

HRMS (ESI): The calculated value of m/z $[M+Na]^+$ was 903.48304, and the measured value was 903.48968.

HPLC: Analysis with an YMC-Pack C18 column (250 mm × 4.6 mm, particle diameter of 5 μm, flow rate of 0.7 ml/min, solvent; $CH_3CN$:$H_2O$=1:1) revealed elution at 27.2 min.

UVmax = 270(s), 318(w), 407(w) nm (50% EtOH)

Synthesis example 1

Synthesis of Glu-TC11

Step 1

Aceto-glycosylation of hydroxyethoxy-1a

Hydroxyethoxy-la (309.1 mg, 0.749 mmol) was weighed in a 50-ml two-necked eggplant flask, dried under vacuum for about 1 hour, and then dissolved in dehydrated acetonitrile (4.5 ml). Diisopropylethylamine (256.6 μl, 1.50 mmol) and acetobromo-α-D-glucose (616.9 mg, 1.50 mmol) weighed in a 20 ml pear-shaped flask and dissolved in dehydrated acetonitrile (5.5 ml) were added, followed by heating under reflux at 80°C for a whole day and night. The reaction solution was transferred into a 50-ml eggplant flask and then evaporated to dryness, thereby obtaining 1.15 g of a residue. The residue was dissolved in 2.5 ml of chloroform, and then injected into the upper part of a silica gel column (ϕ3.5 × 10.5 cm; 100.8 ml) subjected to swelling in chloroform. After washing with chloroform:ethyl acetate (90:1), a target reaction product was separated with chloroform:ethyl acetate (80:1).

[Chemical formula 18]

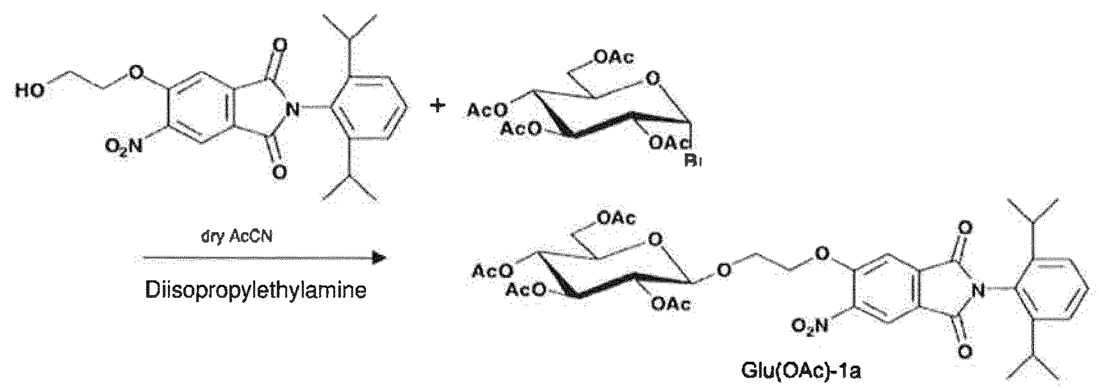


Glu(OAc)-1a

The results of $^1$H-NMR (deuterated chloroform) confirmed signals derived from glucose and from an acetyl group and that the collected reaction product was Glu(OAc)-1a. However, TLC results revealed the presence of substances suspected of coming from glucose. The yield was 739.5 mg (0.939 mmol, about 100%).

The Rf values of silica gel TLC (developing solvent: chloroform/ethyl acetate = 5:1): The Rf value of the raw material, hydroxyethoxy-la, was 0.33, the same of $\alpha$-acetobromo glucose was 0.63, and the same of the product, Glu(OAc)-1a, was 0.50 (positive for p-anisaldehyde reaction for confirmation of sugar chain).

$^1$H NMR (400 MHz, $CDCl_3$): $\delta$8.29 (s, 1H), 7.65 (s, 1H), 7.48 (t, J =7.8 Hz, 1H), 7.30 (d, J =7.8Hz, 2H), 5.77 (d, J =5.2 Hz, 1H), 5.22 (t, J =7.4 Hz,1H), 4.93 (dd, J =2.4, 8.4 Hz, 1H), 4.40 (m, 1H), 4.39 (m, 2H), 4.21 (d, J =4.2 Hz, 2H), 3.95 (t, J =4.4 Hz, 2H), 3.93 (m, 1H), 2.65 (sep, J =6.8 Hz, 1H), 2.64 (sep, J =6.8 Hz, 1H), 2.22 (s, 3H), 2.11 (s, 3H), 2.10 (s, 3H), 1.77 (s, 3H), 1.15 (d, J =7.2 Hz, 12H).

Step 2

Synthesis of Glu(OAc)-TC11 (hydrogenation reaction of Glu(OAc)-1a)

Glu(OAc)-1a (739.5 mg, 0.939 mmol) was added to a 300-ml two-necked eggplant flask, and then dissolved in 100 ml of tetrahydrofuran:ethanol (1:1). After addition of 5% Pd/C (460.9 mg) under an argon gas flow, substitution with a hydrogen gas was performed, and then stirring was performed for a whole day and night at room temperature. The reaction solution was filtered using sellite, and then the filtrate was evaporated to dryness.

[Chemical formula 19]

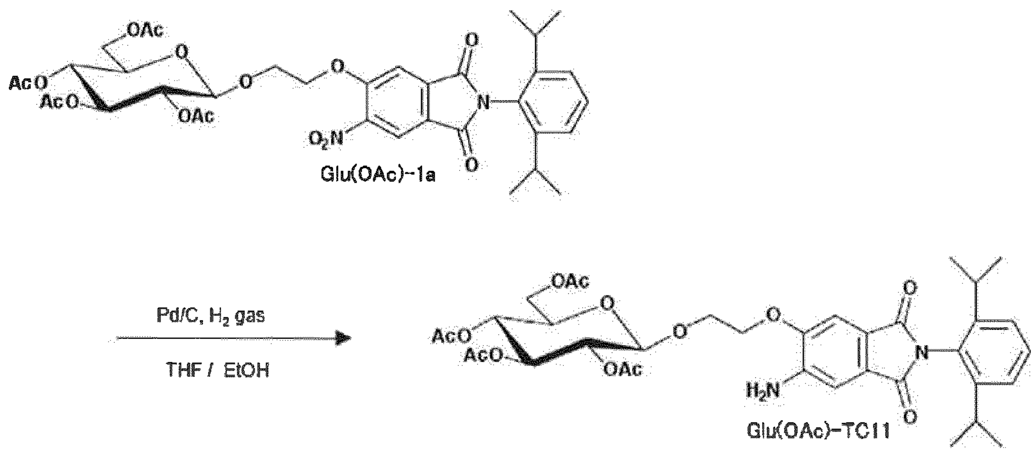


Glu(OAc)-1a

Glu(OAc)-TC11

A residue (679.5 mg) (0.897 mmol, 95.5%) was obtained by the above step. The results of $^1$H-NMR (deuterated

chloroform) confirmed that the reaction product was Glu(OAc)-TC11. TLC results confirmed a spot at Rf=0.40, which was considered to be of Glu(OAc)-TC11. Furthermore, few spots having pale UV and fluorescence at Rf=0.52 and not reacting with anise were observed. The residue was directly used for deacetylation reaction.

The Rf values of silica gel TLC (developing solvent: chloroform/ethyl acetate = 5:1): The Rf value of Glu(OAc)-1a was 0.50, and the same of the product, Glu(OAc)-TC11, was 0.40. In addition, both spots were found to be positive for p-anisaldehyde reaction for confirmation of sugar chain.

$^1$H NMR (400 MHz, $CDCl_3$): δ7.29 (s, 1H), 7.15 (s, 1H), 7.42 (t, J =7.8 Hz, 1H), 7.26 (d, J =7.8Hz, 2H), 5.73 (d, J =5.2 Hz, 1H), 5.20 (t, J =2.8 Hz,1H), 4.91 (dd, J =2.4, 10.0 Hz, 1H), 4.35 (dd, J =2.8, 5.6Hz, 1H), 4.29 (m, 2H), 4.21 (d, J =4.0 Hz, 2H), 3.97 (m, 1H), 3.93 (t, J =4.6Hz, 2H), 2.75 (sep, J =6.8 Hz, 1H), 2.74 (sep, J =6.8 Hz, 1H), 2.10 (s, 9H), 1.79 (s, 3H), 1.15 (d, J =7.2 Hz, 12H).

Step 3

Synthesis of Glu-TC11 by deacetylation of Glu(OAc)-TC11

Glu(OAc)-TC11 (679.5 mg, 0.897 mmol) was added to a 100-ml two-necked eggplant flask, and then dissolved in dehydrated methanol (25 ml). Trimethylamine (5.6 ml, 40.2 mmol) was added and then the mixture was stirred for a whole day and night at room temperature. The reaction solution was dissolved in methanol, transferred into a 100-ml eggplant flask, and then evaporated to dryness. Subsequently, toluene was added (not dissolved) and then the resultant was evaporated twice to dryness, thereby removing triethylamine. Furthermore, evaporation to dryness was repeated 3 times with ethyl acetate.

[Chemical formula 20]

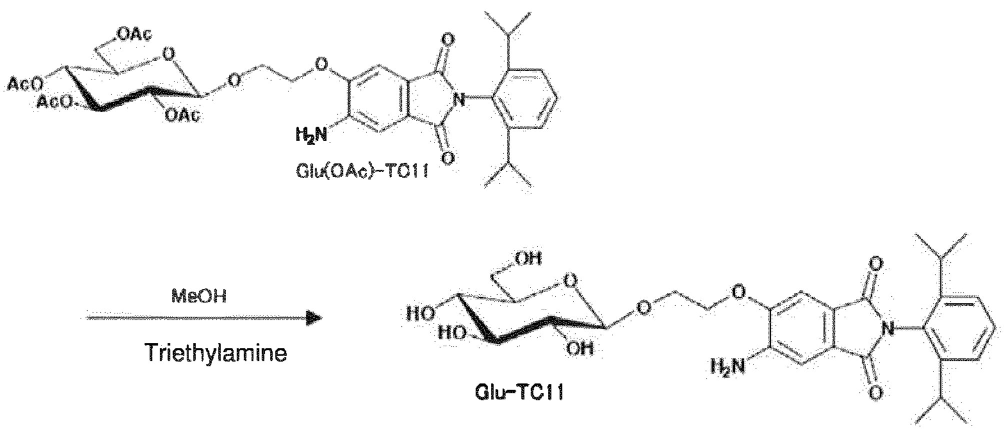


A residue (566.4 mg) was obtained by the above step. $^1$H-NMR (deuterated chloroform) results revealed disappearance of the signal of the acetyl group, so that the reaction product was inferred to be Glu-TC11. Next, the reaction product was purified with a silica gel column.

The residue was dissolved in 1.5 ml of chloroform, and then injected into the upper part of a silica gel column (ϕ2.7 × 11 cm; 62.7 ml) subjected to swelling in chloroform. After washing of the column with chloroform, a compound corresponding to Rf=0.65 was eluted with chloroform:methanol (98:2). Furthermore, after application of chloroform:methanol (97:3), a reaction product corresponding to Rf=0.48 was eluted with chloroform:methanol (96:4), thereby removing the solvent by evaporation.

A yellow solid was obtained by the above step. As a result of $^1$H-NMR spectroscopy in deuterated chloroform, the compound with Rf=0.48 was identified as Glu-TC11. The compound corresponding to Rf=0.65 was identified as hydroxyethoxy-TC11. The yield was 384.4 mg (0.652 mmol), and the yield (%) was 87% relative to the starting material, hydroxyethoxy-la.

The Rf values of silica gel TLC (developing solvent: chloroform/ethyl acetate =5:1): The Rf value of Glu(OAc)-TC11 was 0.77, and the same of the product, Glu-TC11, was 0.48. In addition, both spots were positive for p-anisaldehyde reaction for confirmation of sugar chain.

$^1$H NMR (400 MHz, $CD_3OD$): δ7.35 (s, 1H), 7.45 (t, J =7.8 Hz, 1H), 7.29 (d, J =7.8Hz, 2H), 7.15 (s, 1H), 5.76 (d, J =5.2 Hz, 1H), 4.32 (t, J =4.4 Hz, 2H), 4.29 (t, J =4.8 Hz, 1H), 3.96 (dd, J =4.4, 7.6 Hz, 2H), 3.84 (t, J =4.6 Hz,1H), 3.80 (dd, J =2.4, 12.0 Hz, 1H), 3.68 (dd, J =5.6, 12.0 Hz,1H), 3.60 (ddd, J =2.4, 5.6, 8.8 Hz, 1H), 3.49 (dd, J =4.8, 9.6Hz, 1H),

2.72 (sep, J =6.9 Hz, 2H), 1.14 (d, J =6.9 Hz, 12H).
HPLC: Analysis with an YMC-Pack C18 column (250 mm × 4.6 mm, particle diameter of 5 μm, flow rate of 0.7 ml/min, solvent; $CH_3CN$:$H_2O$=1:1) revealed elution at 12.8 min.
UVmax = 268(s), 316(w), 400(w) nm (50% EtOH)

Example 4

Synthesis of hydroxyethoxy-TC11 (HOEtO-TC11)

Step 1

Synthesis of hydroxyethoxy-TC11 by hydrogenation reaction of hydroxyethoxy-1a

Hydroxyethoxy-la (220.9 mg, 0.536 mmol) was added to a 200-ml two-necked eggplant flask, and then dissolved in 30 ml of tetrahydrofuran:ethanol (1:1). After addition of 5% Pd/C (294.2 mg) under an argon gas, substitution with a hydrogen gas was performed, and then stirring was performed for a whole day and night at room temperature. The reaction solution was filtered using sellite, and then the filtrate was evaporated to dryness, thereby obtaining 243.5 mg of a dark green powder. The powder was dissolved in 30 ml of chloroform, and then injected into the upper part of a silica gel column (ϕ3.5 cm × 6 cm; 57.6 ml) subjected to swelling in chloroform. After washing sequentially with chloroform: ethyl acetate (100), (50:1), and then (20:1), and then a target reaction product was eluted with chloroform:ethyl acetate (5:1).

[Chemical formula 21]

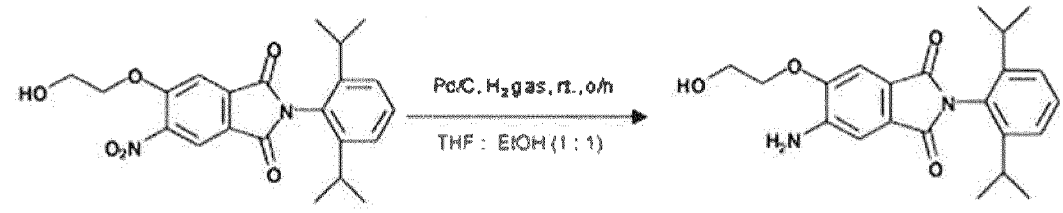


Hydroxyethoxy-1a

Hydroxyethoxy-TC11

As a result of $^1$H-NMR spectroscopy in deuterated chloroform, the reaction product was identified as hydroxyethoxy-TC11. Silica gel TLC results confirmed the spot of only hydroxyethoxy-TC11. The yield was 199.2 mg (0.521 mmol, 97.2%).
The Rf values of silica gel TLC (developing solvent: chloroform/ethyl acetate = 1:1): The Rf value of the raw material, hydroxyethoxy-la, was 0.63, and the same of hydroxyethoxy-TC11 was 0.2.
$^1$H NMR (400 MHz, $CDCl_3$): δ7.43 (t, J =7.8 Hz, 1H), 7.34 (s, 1H), 7.27 (d, J =7.8 Hz, 2H),7.18 (s, 1H), 4.29 (t, J =4.3 Hz, 2H), 4.07 (m, 2H), 2.73 (sep, J =6.8 Hz, 2H), 1.16 (d, J =6.8 Hz, 12H).
HPLC: Analysis with an YMC-Pack C18 column (250 mm × 4.6 mm, particle diameter of 5 μm, flow rate of 0.7 ml/min, solvent; $CH_3CN$: $H_2O$=1:1) revealed elution at 8.1 min.

UVmax = 269(s), 321(s), 311(w), 414(w) nm (50% EtOH)

Example 5

Evaluation of water solubility of drugs

Excess amounts of TC11, PEG5(E)-TC11, HOEtO-TC11, Glu-TC11, PEG11(E)-TC11, and PEG11(0)-TC11 were each added to 100 mM Tris-HCl (pH 7.5), the solutions were stirred at 25°C, the supernatant of each solution was collected every 5 hours, and then the concentration was measured using high performance liquid chromatography (YMC-Pack C18 column (250 mm × 4.6 mm, particle diameter of 5 μm, flow rate of 0.7 ml/min, solvent; $CH_3CN$: $H_2O$=1:1). A point, at which the concentration was constant, was designated as a saturation concentration. Table 1 shows the results.

[Table 1]

Table 1 Water solubility of various drugs

| Drug | g/100ml 100 mM Tris-HCl (pH 7.5) |
|---|---|
| TC11 | 0.002 (×1) |
| PEG5(E)-TC11 | 0.002 (×1) |
| HOEtO-TC11 | 0.027 (×13.5) |
| Glu-TC11 | 0.082 (×41) |
| PEG11(O)-TC11 | 1.470 (×735) |
| PEG11(E)-TC11 | 8.894 (×4,447) |
| Numerals in parentheses indicate relative ratios | |

The results in Table 1 revealed that HOEtO-TC11 had solubility 13.5 times, Glu-TC11 had solubility 41 times, PEG11(E)-TC11 had solubility 4,447 times, and PEG11(O)-TC11 had solubility 735 times improved than that of TC11. As a result, PEG11(E)-TC11 having solubility 4000 times improved than that of TC11 enables more various methods of administration than that in the case of TC11, including peroral administration, intravenous injection, hypodermic injection, intramuscular injection, and the like.

Example 6

Stability of PEG11(E)-TC11

First, PEG11(E)-TC11(A) and PEG11(O)-TC11(B) were examined for stability in mouse blood. To 5-week-old male ICR mice (CLEA Japan, Tokyo, Japan), PEG11(E)-TC11 and PEG11(0)-TC11 were administered intraperitoneally at 59.1 mg/kg BW and 54.6 mg/kg BW, respectively, in such a manner that each molar concentration was equivalent to that of 20 mg/kg BW of TC11. At 0, 0.5, 1, 1.5, 4, 8, 12, and 24 hours later, a tail vein was punctured with a 26G needle for bleeding, and then about 100 μL of blood was collected using a heparinized hematocrit capillary tube (Terumo, Tokyo, Japan). After blood collection, blood was transferred into a 0.5-mL tube, and then subjected to centrifugation at 4°C and 3,400 G for 15 minutes. Subsequently, the supernatant was collected, and then cryopreserved at -80°C. A quality control serum, Consera (registered trademark) (Nissui pharmaceutical, Tokyo, Japan) was dissolved in 3 mL of distilled water ($dH_2O$) per bottle. After left to stand for 30 minutes, bottles were turned upside-down (mixing by inversion) and then used. Consera (190 μL) was mixed with the collected blood plasma and 10 μL of urine, thereby preparing samples. Moreover, PEG11(E)-TC11 and HOEtO-TC11 were each diluted with $dH_2O$ in such a manner that the concentrations were 0, 2, 4, 6, 8, and 10 μM, and then 10 μL of the solution at each concentration and 190 μL of Consera were mixed, thereby preparing a calibration curve sample. Sep-Pak (registered trademark) C18 solid phase extraction cartridge (Waters Associates, Massachusetts, USA) was washed with 2 mL of 100% $CH_3CN$, and then equilibrated with 4 mL of $dH_2O$. Each sample and a sample for preparing a calibration curve were added to the cartridge, and then rinsed with 200 μL of $dH_2O$. Subsequently, the resultant was washed with 2 mL of $dH_2O$, 2 mL of 40%$CH_3CN$, and 250 μL of 100% $CH_3CN$, and then eluted with 1 mL of 100% $CH_3CN$. The eluate was evaporated under vacuum using a centrifugal evaporator CVE-2000 (TOKYO RIKAKIKAI CO, LTD, Tokyo, Japan), redissolution was performed with 100 μL of 100% EtOH, and then filtration was performed using a chromatodisc having a pore size of 0.45 μm (GL sciences, Tokyo, Japan). The high performance liquid chromatography (High Performance Liquid Chromatography; HPLC) apparatuses used herein are the following apparatuses manufactured by JASCO (Tokyo, Japan).

| | |
|---|---|
| Intelligent HPLC pump | PU-2089 |
| Autosampler | AS-2057 |
| Column oven | Co-2060 |
| UV detector | UV-2075 |
| Fluorescence detector | FP-2020 |
| Chromatography data station | LC-NetII/ADC |

HPLC analysis was conducted using Inertsil (registered trademark) ODS-3 250 nm×Φ4.6 mm as a stationary phase, $CH_3CN:dH_2O$=50:50 as a mobile phase, a flow rate of 0.70 mL/min, a UV detection wavelength of 267 nm, and an excitation wavelength of 380 nm, a fluorescence wavelength of 530 nm, and an injection amount of 10 μL at a column temperature of 25°C.

Unlike TC11, no residual drug was observed within the abdominal cavity when PEG11(E)-TC11 and PEG11(O)-TC11 were administered. As shown in Fig. 2A, in the case of subjects to which PEG11(E)-TC11 had been administered, no peak was detected at 0.5 hour after administration during the retention time (tR) of PEG11(E)-TC11 itself, but peaks were detected during only the same tR as that of HOEtO-TC11 having a structure from which water soluble substituent, PEG, had left. The results suggest that PEG11(E)-TC11 is quickly hydrolyzed with esterase in blood, and converted to HOEtO-TC11. On the other hand, in the case of subjects, to which PEG11(0)-TC11 had been administered, a clear peak corresponding to PEG11(0)-TC11 was observed at 0.5 hour after administration, and even 1.5 hours later, a peak was observed at the same position (Fig. 2B). The results suggest that PEG11(O)-TC11 was not degraded with esterase in blood and was not converted to another compound.

Subsequently, PEG11(E)-TC11 was examined for stability in mouse blood plasma (A) and in medium (B). As shown in Fig. 3A, it was revealed that PEG11(E)-TC11 is very quickly hydrolyzed by esterase in mouse blood plasma, and converted to HOEtO-TC11. Moreover, as shown in Fig. 3B, PEG11(E)-TC11 was slowly degraded even in medium and then converted to HOEtO-TC11. Such degradation is considered to result from a small amount of esterase in FBS (fetal calf serum) contained in the medium.

Example 7

*In vitro* cell proliferation inhibitory activity of TC11 and its derivative against high-risk MM cell lines

For examination of cell proliferation inhibitory activity, high-risk MM cell lines (KMM1, KMS11, KMS21, KMS26, KMS28, KMS34) established by Takemi Otsuki et al., of Kawasaki Medical School were used (Otsuki, T. et al., Int. J. Oncol., 15: 1205-1212, 1999). MM cell line MUM24 was established by one of the present inventors, Yutaka Hattori by himself from patients resistant to thalidomide therapy (Hattori, Y. et al., Blood Cancer J., 3: e115, 2013). The genetic abnormality of these cell types, and in particular deletion of p53 gene were examined by FISH method (Chang, H. Blood, 105: 358-360, 2005) (WO2010/ 061862A1). As a result, KMM1, KMS11, KMS26, KMS28, KMS34, and MUM24 were found to have one p53 gene-deficient allele, and KMS21 was found to have alleles with no p53 deletion. These high-risk MM cell lines were treated with drugs such as TC11, HOEtO-TC11, PEG11(E)-TC11, and PEG11(O)-TC11 at arbitrary concentrations. Cell viability after 48 hours was determined using an MTT assay method (Mosman, T. J. Immunol. Methods, 65, 55-63, 1983), thereby calculating $IC_{50}$ values. Specifically, each high-risk MM cell line was prepared at a concentration of $2\times10^5$ cells/ml, and then seeded in an amount of 50 μl ($1\times10^4$ cells) per well. After 24 hours, drugs such as TC11, HOEtO-TC11, PEG11(E)-TC11, and PEG11(O)-TC11 having arbitrary concentrations were added. At 48 hours after addition of each drug, 10 μL each of an MTT labeling reagent in Cell Proliferation Kit I [MTT] (Roche Diagnostics, Basel, Switzerland) was added. After 4 hours of treatment at 37°C, 100 μL each of solution buffer was added and then left to stand overnight at 37°C. Absorbance was measured using a microplate reader Infinit™ M1000 (Tecan, Maennedorf, Switzerland) at a wavelength of 570 nm. Cell viability was found from an absorbance ratio using absorbance at 0 μM designated as 100%. $IC_{50}$ was calculated from a semi-logarithmic chart using Microsoft (registered trademark) Excel.

As shown in Table 2, drugs such as TC11, HOEtO-TC11, PEG11(E)-TC11, and PEG11(O)-TC11 exhibited significant cell proliferation inhibitory activity against many high-risk MM cell lines. Particularly, PEG11(E)-TC11 had a high drug effect, and its $IC_{50}$ value ranged from 1 to 7 μM, the level of which was almost the same as TC11's $IC_{50}$ value of 2-8 μM. PEG11(O)-TC11 also exhibited significant cell proliferation inhibitory activity, but the drug effect ($IC_{50}$ value of 5-60 μM) varied for different cell lines. PEG11(0)-TC11 had an $IC_{50}$ value 5 to 10 times higher and a drug effect lower than those of PEG11(E)-TC11. HOEtO-TC11 had also a high drug effect ($IC_{50}$ value of 1-8 μM), the level of which was almost the same as that of TC11 or PEG11(E)-TC11. It was inferred from these results as follows. PEG11(E)-TC11 is hydrolyzed by esterase in medium and within cells for conversion thereof to PEG11(E)-TC11 having a small molecular size, and then finally starts to act. Hence, unlike PEG11(0)-TC11 that has a large molecular size and thus is not degraded within cells, PEG11(E)-TC11 has high reactivity with a target molecule in view of molecular mobility and thus has a high drug effect.

Moreover, the concentration dependency of the *in vitro* cell proliferation inhibitory activity of TC11, and its derivatives, PEG11(E)-TC11 and HOEtO-TC11 against high-risk MM cell line (KMS21) was examined in detail (Fig. 4). The 3 μM (near the $IC_{50}$ values of TC11, HOEtO-TC11, and PEG11(E)-TC11) of PEG11(E)-TC11 and the same of HOEtO-TC11 exhibited proliferation inhibitory activity against KMS21 cells, nearly 3 to 4 times higher than that of TC11 as shown in Table 2. Such a phenomenon was similarly observed for other high-risk MM cell lines, KMS11, KMS34, and MUM24. The possible reasons why the 3 μM (near the $IC_{50}$ value) of PEG11(E)-TC11 and HOEtO-TC11 exhibited

proliferation inhibitory activity extremely higher than that of TC11 are that: (1) the activity of incorporation of the drugs into cells was improved because of improved water solubility of the drugs; and (2) PEG11(E)-TC11 was hydrolyzed by intracellular carboxyesterase and then finally converted to HOEtO-TC11, and affinity between HOEtO-TC11 and a drug target was higher than that between TC11 and the same, and thus the drug effect was improved.

[Table 2]

Table 2 *In vitro* cell proliferation inhibitory activity of TC11 and TC11 derivatives against high-risk MM cell lines

| | Chromosome aberration | TC11 | HOEtO-TC11. | PEG11(E)-TC11 | PEG11(O)-TC11 |
|---|---|---|---|---|---|
| KMM1 | t(6;14), t(8;14), del17 | 5.1 | 7.7 | 6.3 | 59.3 |
| KMS11 | t(4;14), del17 | 2.0 | 3.9 | 1.4 | 4.8 |
| KMS21 | t(11;14) | 2.8 | 2.4 | 1.6 | 22.7 |
| KMS26 | t(4;14), t(4;16), del1 7 | 3.0 | 1.9 | 2.7 | 16.4 |
| KMS28 | t(4;14), del17 | 7.1 | | 6.8 | 25.8 |
| KMS34 | t(4;14), del17 | 4.0 | 4.1 | 2.6 | 10.5 |
| MUM24 | t(4;14), del13, del17 | 3.0 | 4.0 | 1.8 | 9.2 |
| Numerical values indicate $IC_{50}$ (μM) | | | | | |

Example 8

Similar to Example 7, high-risk MM cell lines were treated with drugs, TC11 and PEG5(E)-TC11 at arbitrary concentrations. Cell viability after 48 hours was determined using a WST-1 assay method (Cook, JA. & Mitchell, JB., Anal. Biochem., 179, 1-7, 1984), and thus $IC_{50}$ values were calculated. Specifically, each high-risk MM cell line was prepared at $5\times10^5$ cells/ml, and then seeded at 50 μl per well ($2.5\times10^4$ cells). Drugs such as TC11, and PEG5(E)-TC11 were added to wells at arbitrary concentrations. WST-1 reagent was added at 48 hours after addition of drugs. After 1 hour of reaction at 37°C in a $CO_2$ incubator, absorbance was measured at a wavelength of 450 nm using a microplate reader. Cell viability was found from an absorbance ratio using absorbance at 0 μM designated as 100%. $IC_{50}$ was calculated from a semi-logarithmic chart using Microsoft (registered trademark) Excel.

As shown in Table 3, drugs such as TC11 and PEG5(E)-TC11 exhibited significant cell proliferation inhibitory activity against various high-risk MM cell lines, and the drug effect ($IC_{50}$ value of 1.3-4.6 μM) of PEGS(E)-TC11 was almost the same as the $IC_{50}$ value of TC11.

[Table 3]

Table 3 *In vitro* cell proliferation inhibitory activity of TC11 and PEG5(E)-TC11 against high-risk MM cell lines

| MM cell type | Chromosome aberration | TC11 | PEG5(E)-TC11 |
|---|---|---|---|
| KMM1 | t(6;14). t(8;14), del17 | 5.7 | 3.6 |
| KMS11 | t(4;14), del17 | 3.2 | 1.4 |
| KMS21 | t(11;14) | 1.6 | 1.2 |
| KMS34 | t(4;14), del17 | 6.5 | 6.5 |
| Numerical values indicate $IC_{50}$ (μM) | | | |

Example 9

*In vitro* cell proliferation inhibitory activity of PEG11(E)-TC11 and lenalidomide against various high-risk MM cells

MM cell lines (KMM1, KMS11, KMS21, KMS26, KMS27, KMS28, KMS34) used herein were those established by Takemi Otsuki et al., of Kawasaki Medical School (Otsuki, T. et al., Int. J. Oncol., 15: 1205-1212, 1999). MUM24 used herein was established by one of the present inventors, Yutaka Hattori by himself from patients resistant to thalidomide therapy (Hattori, Y. et al., Blood Cancer J., 3: e115, 2013). These cell lines were each treated with 0-30μM PEG11(E)-TC11 and 0-100 μM lenalidomide, respectively, and then 48 hours later, cell viability was calculated in a manner similar to that in Example 7 using the MTT assay method (Mosman, T. J. Immunol. Methods, 65, 55-63, 1983).

As shown in Fig. 5A, PEG11(E)-TC11 exhibited significant *in vitro* cell proliferation inhibitory activity against various high-risk MM cells, however, lenalidomide did not exhibit *in vitro* cell proliferation inhibitory activity, against myeloma cells other than KMS21 and MUM24, including high-risk MM cells, KMS11, KMS28, and KMS34, as shown in Fig. 5B. These results successfully confirmed that PEG11(E)-TC11 is very effective, but lenalidomide is not significantly effective to inhibit high-risk MM cell proliferation.

Example 10

*In vitro* cell proliferation inhibitory activity of PEG11(E)-TC11 and PEG11(O)-TC11 against various solid cancer cells

Cancer cells used herein were KMS34 (human myeloma cells), HeLa (human cervical cancer cells), HCT116 (human colon cancer cells; p53+/+), KATOIII (human stomach cancer cells), MDA-MB-231 (human breast cancer cells), HT-29 (human colon adenocarcinoma cells), PANC-1 (human pancreatic cancer cells), and Li-7 (human liver cancer cells). Proliferation assay of various cancer cells was performed by the WST-1 method (Cook, JA. & Mitchell, JB., Anal. Biochem., 179, 1-7, 1984) as follows. One μl each of PEG11(E)-TC11 and PEG11(O)-TC11 prepared in advance at target concentrations was added into each well of a 96-well microplate. Medium was added to the wells at 50 μl per well, and then mixed by tapping lightly the plate. Subsequently, KMS34 was prepared at a concentration of $5\times10^5$ cells/ml, and cells other than KMS34 were prepared at a concentration of $2\times10^5$ cells/ml. Cells were seeded at 50 μl per well ($2.5\times10^4$ cells, $1\times10^4$ cells), and then incubated in a $CO_2$ incubator (37°C, 5% $CO_2$) for 48 hours. Ten μl each of cell proliferation reagent WST-1 (Roche) was added to each well after incubation, and then the resultants were mixed by lightly tapping the plate, followed by 1 hour of reaction in the $CO_2$ incubator. Subsequently, the plate was shaken with a plate reader for 1 minute, and then absorbance was measured at 450 nm. At this time, absorbance was also measured at 595 nm as a reference wavelength. The net absorbance was obtained by subtracting the absorbance at the reference wavelength from the absorbance at 450 nm.

[Table 4]

Table 4 *In vitro* cell proliferation inhibitory activity of PEG11 (E)-TC11 and PEG11 (O)-TC11 against various cancer cells

| Drug | KMS34 | HeLa | HCT116 | KATOIII | MDA-MB-231 | HT-29 | PANC-1 | Li-7 |
|---|---|---|---|---|---|---|---|---|
| PEG11 (E))-TC11 | 0.67 | 5.4 | 9.0 | 7.9 | 11.0 | 20.5 | 14.0 | 9.4 |
| PEG11 (O)-TC11 | 16.0 | 18.1 | 34.5 | 21.0 | 80.0 | 11.4 | 75.0 | 87.0 |
| TC11 | 2.6 | - | - | - | - | - | - | - |
| Numerical values indicate $IC_{50}$ (μM) | | | | | | | | |

The results in Table 4 revealed that PEG11(E)-TC11 and PEG11(O)-TC11 exhibit *in vitro* cell proliferation inhibitory activity against various cancer cells. It could be confirmed that PEG11(E)-TC11 exhibits a significant drug effect ($IC_{50}$ value of 0.67 μM) on cancer cells and particularly KMS34 (myeloma cells), and also exhibits strong cell proliferation inhibitory activity against HeLa (human cervical cancer cells), KATOIII (human stomach cancer cells), and HCT116 (human colon cancer cells).

Example 11

*In vitro* cell proliferation inhibitory activity of PEG11(E)-TC11 and TC11 against high-risk MM cells (KMS34)

and bone marrow stromal cell-derived normal cells

Proliferation assay of cancer cells and normal cells was performed using WST-1 as follows. One μl each of PEG11(E)-TC11 and TC11 prepared at target concentrations was added to each well of a 96-well microplate. Medium was added to the wells at 50 μl per well, and then mixed by tapping lightly the plate. Next, high-risk MM cells (KMS34) prepared at $5\times10^5$ cells/ml and bone marrow stromal cell-derived normal cells (WT) prepared at $2\times10^5$ cells/ml were seeded at 50 μl ($2.5\times10^4$ cells and $1\times10^4$ cells, respectively) per well, and then incubated in a $CO_2$ incubator (37°C, 5% $CO_2$) for 48 hours. Ten (10) μl each of cell proliferation reagent WST-1 (Roche) was added to each well after incubation, and then the resultants were mixed by lightly tapping the plate, followed by 1 hour of reaction in the $CO_2$ incubator. Subsequently, the plate was shaken with a plate reader for 1 minute, and then absorbance was measured at 450 nm. At this time, absorbance was also measured at 595 nm as a reference wavelength. The net absorbance was obtained by subtracting the absorbance at the reference wavelength from the absorbance at 450 nm.

As shown in Fig. 6, PEG11(E)-TC11 and TC11 each exhibited significant proliferation inhibitory activity against high-risk MM cells (KMS34), but exhibited no proliferation inhibitory activity against bone marrow stromal cell-derived normal cells (WT). These results successfully confirmed that PEG11(E)-TC11 is a drug exhibiting cell proliferation inhibitory activity in a cancer-specific manner against various cancer cells including MM cells.

Example 12

Apoptosis induction of high-risk MM cells (MUM24) by TC11 and PEG11(E)-TC11

For observation of apoptosis induction caused by drugs, TC11 and PEG11(E)-TC11, one type of MM cells, MUM24 cells were treated with TC11 and PEG11(E)-TC11, Annexin V/PI staining was performed, and then analysis was conducted using a flow cytometer. More specifically, MUM24 was adjusted at $2.0\times10^5$ cells/mL, and then 1.95 mL each thereof was seeded to a 6-well plate. From the next day, TC11 and PEG 11 (E)-TC11 were added in such a manner that the final concentrations were 0, and 10 μM, and then cells were cultured at 37°C for 24, 48, and 72 hours. Each cell (1.5 ml each) was collected, and then washed twice with Phosphate Buffered Saline (PBS, Sigma-Aldrich). Subsequently, each resultant was suspended again in a solution prepared by adding 5 μL of Annexin V-FITC and 5 μL of Propidium Iodide (PI) to 500 μL of 1 × Binding Buffer in Annexin V-FITC Apoptosis Detection Kit (Bio Vision, California, USA), and then left to stand in the dark for 5 minutes. Fluorescence intensity was measured using BDTM LSR II Flow Cytometer (Becton, Dickinson and company, New Jersey, USA). Wavelengths to be measured were excitation wavelength of 488 nm, and fluorescence wavelengths of 530±15 nm for Annexin V-FITC and 610±10 nm for PI.

As shown in Fig. 7, both TC11 and PEG11(E)-TC11 were confirmed to cause an increase in, an early apoptosis fraction, Annexin $V^+/PI^-$ cells, and an increase in, a late apoptosis fraction, Annexin $V^+/PI^+$ cells. As a result, it was confirmed that TC11 and PEG11(E)-TC11 clearly induce apoptosis in MM cells.

Example 13

Effects of TC11 and PEG 11(E)-TC11 on cell cycle

The present inventors have reported that when HeLa cells of a human cervical cancer-derived cell line are treated with TC11, multipolarization and then multinucleation take place (Shiheido, H. et al., PLoS One, 7: e38878, 2012). The present inventors have also reported in the same paper α-tubulin and NPM1 as binding molecules for TC11. Next, intracellular DNA content was measured using flow cytometry in order to examine the effects of TC11 and its derivative, PEG11(E)-TC11 on cell cycle. More specifically, MUM24 was adjusted at $4.0\times10^5$ cells/mL, 1.95 mL each of MUM24 was seeded to a 6-well plate. From the next day, TC11 and PEG11(E)-TC11 were added in such a manner that the final concentrations were 0 and 3 μM, respectively, and then cells were cultured at 37°C for 12, 24, and 36 hours. Each cell was collected, and then washed with PBS. 70% EtOH (460 ml) was added and then the resultant was left to stand overnight at - 30°C. After washing with PBS, 100 ml of citrate-phosphate buffer was added, and then the resultant was left to stand at room temperature for 30 minutes, thereby eluting fragmented DNA. After elution of the fragmented DNA, 100 ml of PBS, and 10 ml of 1 mg/ml RNase A (heated) (Roche) were added for 30 minutes of reaction at room temperature. Thereafter, 200 ml of PBS, and 10 ml of 1 mg/ml PI were added, and then the mixture was allowed to react in the dark at room temperature for 30 minutes. Fluorescence intensity was measured using BD FACS Calibur (Trademark) (Becton, Dickinson and company, New Jersey, USA). Wavelengths for measurement were an excitation wavelength of 488 nm, and a fluorescence wavelength of 610±10 nm.

As shown in Fig. 8, MUM24 cells treated with TC11 and the same treated with PEG11(E)-TC11 were each found to be composed of a higher proportion of tetraploid cells than that of the control. Therefore, it was successfully confirmed that TC11 and PEG 11 (E)-TC11 induce cell death by causing G2/M phase arrest in high-risk MM cells (MUM24).

Example 14

Verification of interaction of cereblon protein with TC11, its derivative, and thalidomide or lenalidomide using pull-down assay and surface plasmon resonance biosensor

Protein cereblon (cereblon; CRBN) binding as a teratogen to thalidomide has been recently identified (Ito, T. et al., Science, 327: 1345-1350, 2010). As a result of X-ray crystal structure analysis, thalidomide was confirmed to bind to CRBN, with its glutarimide ring part (Chamberlain, PP, et al., Nature Struct. Mol. Biol., 21: 803-810, 2014). TC11 lacks a glutarimide ring serving as a CRBN binding site of thalidomide, lenalidomide or the like, but instead has a benzene ring having a bulky diisopropylphenyl group as a result of substitution, and thus is inferred to bind to CRBN with difficulty. Here we attempted to verify interaction between CRBN and TC11, its derivative, and thalidomide or lenalidomide using a pull-down assay and a surface plasmon resonance biosensor.

First, the interaction of CRBN with thalidomide and the same with TC11 were verified using a pull-down assay. CRBN was prepared as follows. A T7 tag was added 5'-upstream of, and a FLAG tag and a histidine tag were added 3'-downstream of a full-length CRBN gene. The gene was introduced into a pcDNA3.3 expression vector (Invitrogen), thereby constructing a plasmid DNA. Next, a Kozak sequence was added 5'-upstream of the T7 tag using a KOD-Plus-Mutagenesis Kit (TOYOBO). The plasmid DNA was transfected into 293T cells. After 4 days of culture at 37°C with 5% $CO_2$, cells were extracted with a RIPA buffer (Thermo), and then the extracted fraction was purified using Ni Sepharose excel (GE Healthcare). Since the purified fraction contained imidazole, TBS (Nacalai Tesque) was subjected to buffer exchange. A biotinylated existing pharmaceutical product, thalidomide, and TC11 were each bound to streptavidin beads, and then recombinant full-length CRBN prepared by the above method was reacted with the beads at 30°C for 30 minutes. Centrifugation was performed to separate the resultants into a flow-through (F) fraction and a bead (B) fraction. Protein bands corresponding to CRBN were separated by SDS-PAGE, and then detected by Western blot. As shown in Fig. 9A, it was revealed that CRBN bound to thalidomide immobilized on beads, but did not bind to TC11 immobilized on beads. Note that "I" indicates "Input fraction".

Subsequently, the interaction of CRBN with TC11 and the same with its derivatives, PEG11(E)-TC11, PEG11(O)-TC11, and HOEtO-TC11, and thalidomide or lenalidomide were verified using a surface plasmon resonance biosensor. Dissociation constants $K_D$ of TC11 and its derivatives, PEG11(E)-TC11, PEG11(O)-TC11, and HOEtO-TC11, and thalidomide or lenalidomide for CRBN were estimated by analyzing intermolecular interaction using BIACORE™ 3000 (biosensor based on the principle of surface plasmon resonance, GE Healthcare BioScience Corporation). As a ligand (substance to be adhered to a sensor chip) of a BIACORE biosensor, CRBN having the histidine-tagged C terminus prepared by the above method was used. First, CRBN was immobilized on a Sensor Chip NTA. TBS (Nacalai Tesque) was used as a buffer and applied at a flow rate of 5 μl/min. CRBN (10 μl) was injected after buffer exchange for immobilization of CRBN to flow cell 2 and flow cell 4, and thus 7800 RU (response unit) of CRBN bound to each of them. Meanwhile, TC11 and its derivatives, PEG11(E)-TC11, PEG11(O)-TC11, and HOEtO-TC11 and thalidomide or lenalidomide were dissolved as analytes (substances to be applied to a flow system) in TBS (Nacalai Tesque) buffers, and the concentrations were adjusted at 1 μM, 2 μM, and 10 μM. Analysis of intermolecular interaction using BIACORE 3000 was conducted using TBS buffer at a flow rate of 20 μl/min. These analytes were injected, 60 μl each, using FLOWPATH 1,2 or FLOWPATH 3,4 and KINJECT, and then dissociation was measured for 300 seconds. TC11 and its derivatives, PEG11(E)-TC11, PEG11(O)-TC11, and HOEtO-TC11, and thalidomide or lenalidomide were measured using TBS buffers at concentrations of 1 μM, 2 μM, and 10 μM. Analysis was conducted as follows. A value obtained by subtracting the sensorgram of flow cell 1 from the sensorgram of flow cell 2, or, a value obtained by subtracting the sensorgram of flow cell 3 from the sensorgram of flow cell 4 was analyzed by reaction kinetic analysis using analysis software BIA evaluation ver. 4.1 and 1:1 binding model, thereby determining the dissociation constant ($K_D$).

Fig. 9B shows the surface plasmon resonance biosensorgram of the interaction of CRBN with thalidomide and lenalidomide. Gray denotes actual measured data, and black denotes fitting curves. Good fitting curves were obtained for both in this measurement. Thalidomide exhibited a $K_D$ value of 5.66x $10^{-7}$ M for CRBN. Further, lenalidomide exhibited a $K_D$ value of $3.65\times10^{-6}$ M for CRBN. Fig. 9C shows the surface plasmon resonance biosensorgram of the interaction of CRBN with TC11 and its derivatives, HOEtO-TC11, PEG11(E)-TC11, and PEG11(O)-TC11. All compounds were measured at concentrations of 1 μM, 2 μM, and 10 μM. It was revealed in Fig 9C that TC11 and its derivatives, HOEtO-TC11, PEG11(E)-TC11, and PEG11(O)-TC11 never bind to CRBN. Taken together, TC11 and its derivatives, HOEtO-TC11, PEG11(E)-TC11, and PEG11(O)-TC11 do not bind to CRBN directly involved in teratogenicity, unlike drugs such as thalidomide and lenalidomide, and thus is considered to be drugs having almost no teratogenicity.

Example 15

Mouse blood kinetics upon single intraperitoneal administration of TC11 and PEG11(E)-TC11

Mouse blood kinetics upon single intraperitoneal administration of TC11 and PEG11(E)-TC11 was examined. TC11 (60 mg/kg BW) and PEG11(E)-TC11 (177.3 mg/kg BW) in an equimolar amount thereof were administered to six 5-week-old male ICR mice (CLEA Japan, Tokyo, Japan) via single intraperitoneal administration. At 0, 0.25, 0.5, 1, 1.5, 4, 8, 12, and 24 hours later, about 100 $\mu$L of blood was collected via a tail vein using a heparinized hematocrit capillary tube (Terumo, Tokyo, Japan). After blood collection, centrifugation was performed at 4°C and 3,400 G for 15 minutes, 10 $\mu$l of a supernatant was collected, and then cryopreserved at -80°C. The subsequent serum treatment and HPLC analyses of TC11 and PEG11(E)-TC11 and HOEtO-TC11 were performed by the same method as in Example 7. Note that as described in Example 7, since PEG11(E)-TC11 is quickly hydrolyzed by esterase in blood and then converted to HOEtO-TC11, the blood HOEtO-TC11 level was measured.

As a result, as shown in Fig. 10A, the time at which the blood level reached the highest level (tmax), the highest blood level (Cmax), and the serum half-life (t1/2) upon single intraperitoneal administration of TC11 (60 mg/kg BW) were 1.5 hours, 2.6 $\mu$M, and 1.4 hours, respectively. On the other hand, as shown in Fig. 10B, tmax, Cmax, and t1/2 upon single intraperitoneal administration of PEG11(E)-TC11 (177.3 mg/kg BW) were 1.0 hour, 24.4 $\mu$M, and 2.2 hours, respectively, confirming the highest blood level 9 times higher than that of TC11 and the prolonged serum half-life 1.6 times longer than that of TC11.

Example 16

Antitumor effects of TC11, PEG11(E)-TC11, and PEG11(O)-TC11 on KMS11-bearing mice

TC11 and its derivatives were examined for *in vivo* antitumor effects using 6 KMS11 xenograft model mice. KMS11 was administered to 6-week-old ICR/SCID mice at $3\times10^7$ cells/mouse via hypodermic injection. Tumor volume was measured over time. The day on which each tumor volume exceeded 50 $mm^3$ was designated as Day 1 and observation was performed for 14 days. The single dosage of TC11 was 60 mg/kgBW, the intraperitoneal dosages of PEG11(E)-TC11 and PEG11(O)-TC11 were 177.3 mg/kgBW and 163.8 mg/kgBW, respectively, which were equimolar amounts of that of TC11. Since TC11 is hardly dissolved in physiological saline (NaCl 0.9 w/v%) alone, TC11 was dissolved in DMSO, and then diluted with 1.1% Tween 80/physiological saline, so that it had a predetermined concentration. The final composition of the solvent was 10% DMSO + 1% Tween 80/physiological saline. TC11 was almost completely dissolved under the solvent conditions. PEG11(E)-TC11 and PEG11(O)-TC11 were each completely dissolved in physiological saline. Drug administration was performed for two out of every 3 days (Day 1, 2, 4, 5, 7, 8, 10, 11, 12, 13). Mice were sacrificed on Day 14, and then tumor weights were measured and *in vivo* findings were confirmed.

As shown in Fig. 11, in groups to which TC11, PEG11(E)-TC11, and PEG11(O)-TC11 had been administered, respectively, a tendency of significant decreases in tumor volume was confirmed compared to the control. In particular, administration of PEG11(E)-TC11 resulted in a significant decrease in tumor volume compared to TC11, such that 3 mice having tumor sizes almost the same as those at the time of initiation of administration and 1 mouse from which the primary lesion had completely disappeared were observed. Furthermore, no decrease in body weight was observed in the case of the group to which each compound had been administered, compared to the control group, suggesting the low toxicity at a concentration for suppressing tumor growth.

Example 17

Comparison of antitumor effects of TC11, HOEtO-TC11, and lenalidomide on KMS11 cancer-bearing mice

As shown in Fig. 4 in Example 7, the concentration dependency of *in vitro* cell proliferation inhibitory activity of TC11 and HOEtO-TC11 against the high-risk MM cell line (KMS21) was examined specifically. The 3 $\mu$M (near the $IC_{50}$ values of TC11 and HOEtO-TC11) of HOEtO-TC11 exhibited proliferation inhibitory activity against KMS21 cells, which was nearly 3 to 4 times higher than that of TC11. Furthermore, in Example 9, lenalidomide exhibited no *in vitro* cell proliferation inhibitory activity against myeloma cells other than KMS21 and MUM24 including high-risk MM cells KMS11, KMS28, and KMS34 (Fig. 5B). Moreover, it has been reported that about 20% of patients exhibit resistance to lenalidomide. Accordingly, whether or not HOEtO-TC11 had a drug effect higher than that of TC11 *in vivo,* and whether or not lenalidomide exhibited an antitumor effect *in vivo* on high-risk MM cells KMS11 were examined.

In this experiment, TC11, HOEtO-TC11, and lenalidomide were examined for the antitumor effect *in vivo* using 5 KMS11 xenograft model mice. KMS11 was administered to 5-week-old ICR/SCID mice at $2\times10^7$ cells/mouse via hypodermic injection. Tumor volumes were measured over time, and the day on which the tumor volume exceeded 50 $mm^3$ was designated as Day 1 and observation was performed for 9 days. Single dosage of TC11 was 60 mg/kgBW,

the intraperitoneal dosages of HOEtO-TC11 and lenalidomide were 71.1 mg/kg BW and 49.9 mg/kgBW, respectively, which were equimolar amounts of TC11. Stock solutions of TC11, HOEtO-TC11, and lenalidomide were prepared in advance at 60 mg/mL (33% DMSO, 66% Cremorphor), 71.1 mg/mL (33% DMSO, 66% Cremorphor), and 49.9 mg/mL (33% DMSO, 66% Cremorphor), respectively, and then stored at 4°C. On the day of administration, these solutions were diluted 10-fold with physiological saline while stirring with Vortex, and then intraperitoneally administered. Tumor volume was calculated by the following formula.

$$\text{Tumor volume (mm}^3\text{)} = \text{major axis} \times \text{minor axis} \times \text{minor axis} \times \pi/6 = \text{major axis} \times \text{minor axis} \times \text{minor axis} \times 0.5236$$

As shown in Fig. 12, on Day 9, KMS-11 tumor growth was suppressed by administration of TC11 and HOEtO-TC11 to a degree higher than that in control individual mice to which no drug had been administered, resulting in a clear significant difference. On Day 9, administration of HOEtO-TC11 significantly inhibited KMS-11 tumor growth to a level about 1/3 of that of the control, and about a half of that of TC11. The symbol "*" in the figure indicates that the t-test result (significance level 5%) was more significant than that of one control group. Moreover, lenalidomide had an *in vivo* anti-tumor effect clearly weaker than that of HOEtO-TC11.

Example 18

Synthesis of HOEtO-TC11 (another method)

Step 1

[Chemical formula 22]

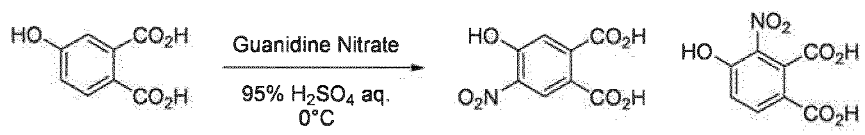


4-Hydroxyfuthalic acid
Mol Wt: 182.13

B
Mol Wt: 227.13

B'

4-hydroxyphthalic acid (21.7 g, 119.0 mmol) was dissolved in 682 mL of 95% concentrated sulfuric acid. The reaction solution was cooled to 5°C, 14.6 g (119.0 mmol) of guanidine nitrate was slowly added for 30 minutes under an argon atmosphere, and then the solution was stirred for 1 hour. The completion of the reaction was confirmed by HPLC, and then the reaction solution was slowly added to cool ice water. After extraction using ethyl acetate, an aqueous layer was further extracted with ethyl acetate. An organic layer was washed with a saturated saline solution, dried using sodium sulfate, and then concentrated under vacuum, thereby obtaining 16.8 g of B + B' as a brown powder (yield 62.1%).

The thus obtained B was a mixture of positional isomers of the nitro group, and the ratio was B : B' = 1 : 1.27.

Step 2

[Chemical formula 23]

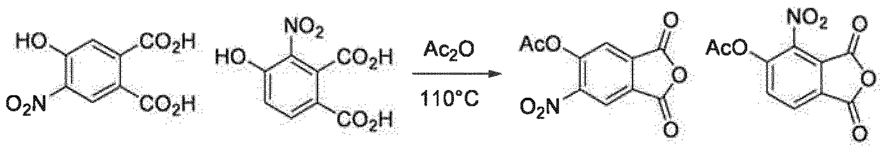


B
Mol Wt: 227.13

B'

D
Mol Wt: 251.15

D'

The mixture of B and B' (22.4 g, 99.0 mmol) was dissolved in 186 mL of acetic anhydride. Under an argon atmosphere, the reaction solution was heated to 110°C and then stirred for 3 hours. The completion of the reaction was confirmed by HPLC, and then the reaction solution was concentrated under vacuum, thereby obtaining 24.8 g of D and D' as a black oil (yield 100%).

The thus obtained D was a mixture of the raw material-derived positional isomers, and the ratio was D : D' = 1 : 1.27.

Step 3

[Chemical formula 24]

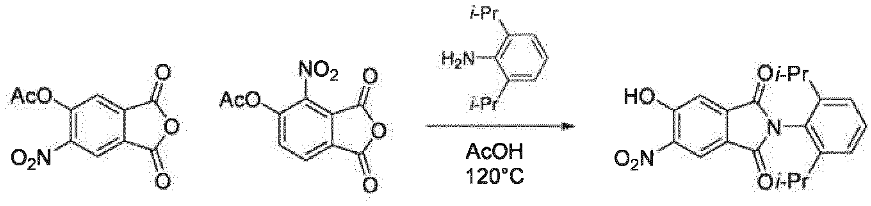


D
Mol Wt: 251.15

D'

1a
Mol Wt: 368.39

The mixture of D and D' (24.8 g, 99.0 mmol) was dissolved in 282 mL of acetic acid, and then 24.0 mL (126.7 mmol) of 2,6-diisopropylaniline was added. The reaction solution was heated to 120°C under an argon atmosphere, and then stirred for 4 hours. Subsequently, 7.8 mL (41.58 mmol) of 2,6-diisopropylaniline was added, and then the mixture was further stirred for 1 hour. The completion of the reaction was confirmed by HPLC, and then the reaction solution was added to ice water. After extraction with ethyl acetate, an aqueous layer was further extracted with ethyl acetate. An organic layer was washed with a saturated saline solution, dried using sodium sulfate, and then concentrated under vacuum. The resultant was purified by silica gel chromatography (heptane/ethyl acetate = 20/1→4/1), and then the main distillate was concentrated under vacuum, thereby obtaining 15.0 g of 1a as a thin yellow powder (two more steps were performed after the preparation of the B+B' mixture, 41.2%).

Step 4

[Chemical formula 25]

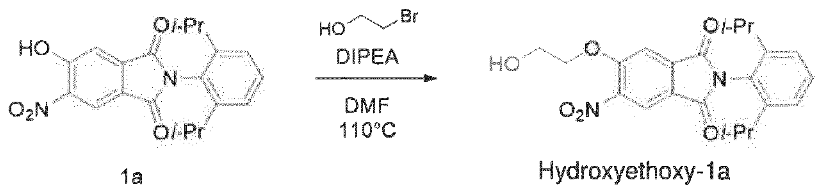


1a
Mol Wt: 368.39

Hydroxyethoxy-1a
Mol Wt: 412.44

1a (13.5 g, 36.6 mmol) was dissolved in 142 mL of DMF, 5.22 mL of 2-bromoethanol (73.3 mmol) and 12.8 mL of DIPEA (73.3 mmol) were added. The reaction solution was heated to 110°C under an argon atmosphere, and then stirred for 18 hours. The completion of the reaction was confirmed by HPLC, and then water was added. After extraction with ethyl acetate, an aqueous layer was further extracted with ethyl acetate. An organic layer was washed with a saturated saline solution, dried using sodium sulfate, and then concentrated under vacuum. The resultant was purified by silica gel chromatography (heptane/ethyl acetate = 50/1→0/1), the main distillate was concentrated under vacuum, and then subjected again to silica gel chromatography (chloroform/ethyl acetate = 1/0→3/1), thereby obtaining 13.2 g of hydroxyethoxy-la as a thin yellow powder (yield 87.3%).

Step 5

[Chemical formula 26]

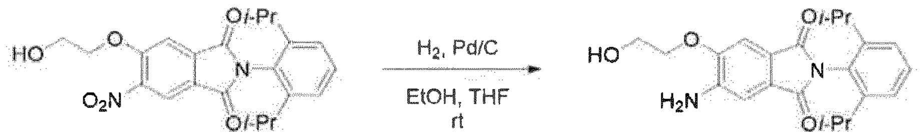


Hydroxyethoxy-1a
Mol Wt: 412.44

Hydroxyethoxy-TC11
Mol Wt: 382.46

Pd/C (K-type) (2.3 g) was added to a solution (mixture of 200 mL of EtOH and 200 mL of THF) of 7.0 g of hydroxyethoxy-la (16.97 mmol), and then the mixture was stirred under a hydrogen atmosphere at room temperature for 3 hours.

The completion of the reaction was confirmed by HPLC, the reaction solution was filtered, and then concentrated under vacuum. The resultant was purified by silica gel chromatography (chloroform/ethyl acetate = 1/0→1/1) and then the main distillate was concentrated under vacuum, thereby obtaining 5.5 g of hydroxyethoxy(HOEtO)-TC11 as a yellow powder (yield: 84.7%).

## Claims

1. A phenylphthalimide derivative represented by the following general formula:

[Chemical formula 1]

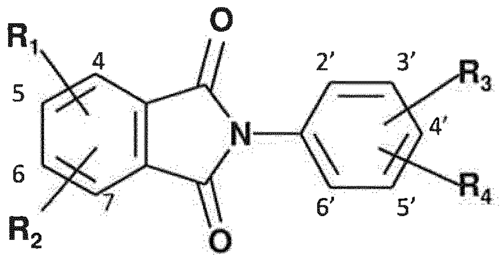

wherein $R_1$ is a group containing PEG or a hydroxy $C_{1-5}$ alkoxy group binding to position 4 or position 5, $R_2$ is an amino group at position 6, and $R_3$ and $R_4$ are each independently a hydrogen atom, an alkyl group, an aryl group, an aralkyl group, or an aryloxy group.

2. The derivative according to claim 1, wherein $R_1$ is a group containing PEG.

3. The derivative according to claim 2, wherein $R_1$ is a group in which PEG binds to a phthalimide ring via an ester bond.

4. The derivative according to claim 1, wherein $R_1$ is a hydroxy $C_{1-5}$ alkoxy group.

5. The derivative according to any one of claims 1 to 4, wherein $R_1$ binds to position 5.

6. The derivative according to any one of claims 1 to 5, wherein $R_3$ and $R_4$ are both isopropyl groups at position 2' and position 6', respectively.

7. A pharmaceutical composition, comprising the derivative according to any one of claims 1 to 6.

**8.** The pharmaceutical composition according to claim 7, which is an anticancer agent.

**9.** The pharmaceutical composition according to claim 8, wherein the anticancer agent is used for multiple myeloma.

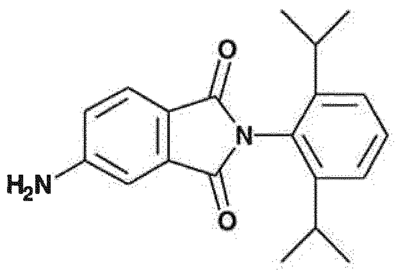

TC11

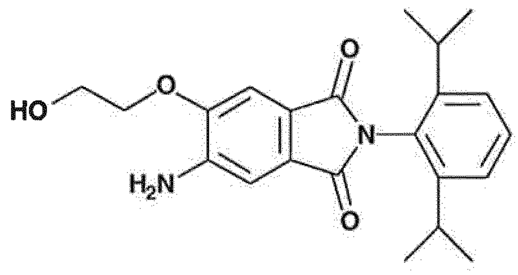

HOEtO-TC11

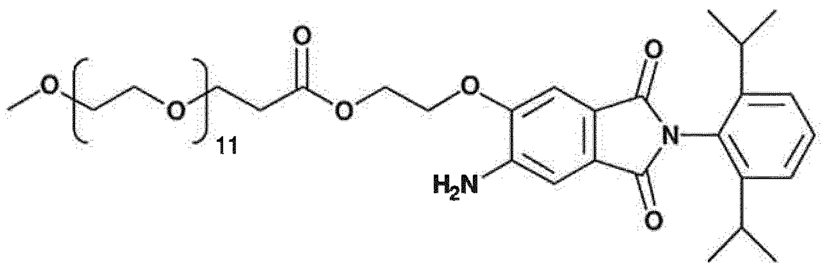

PEG11(E)-TC11

Fig. 1

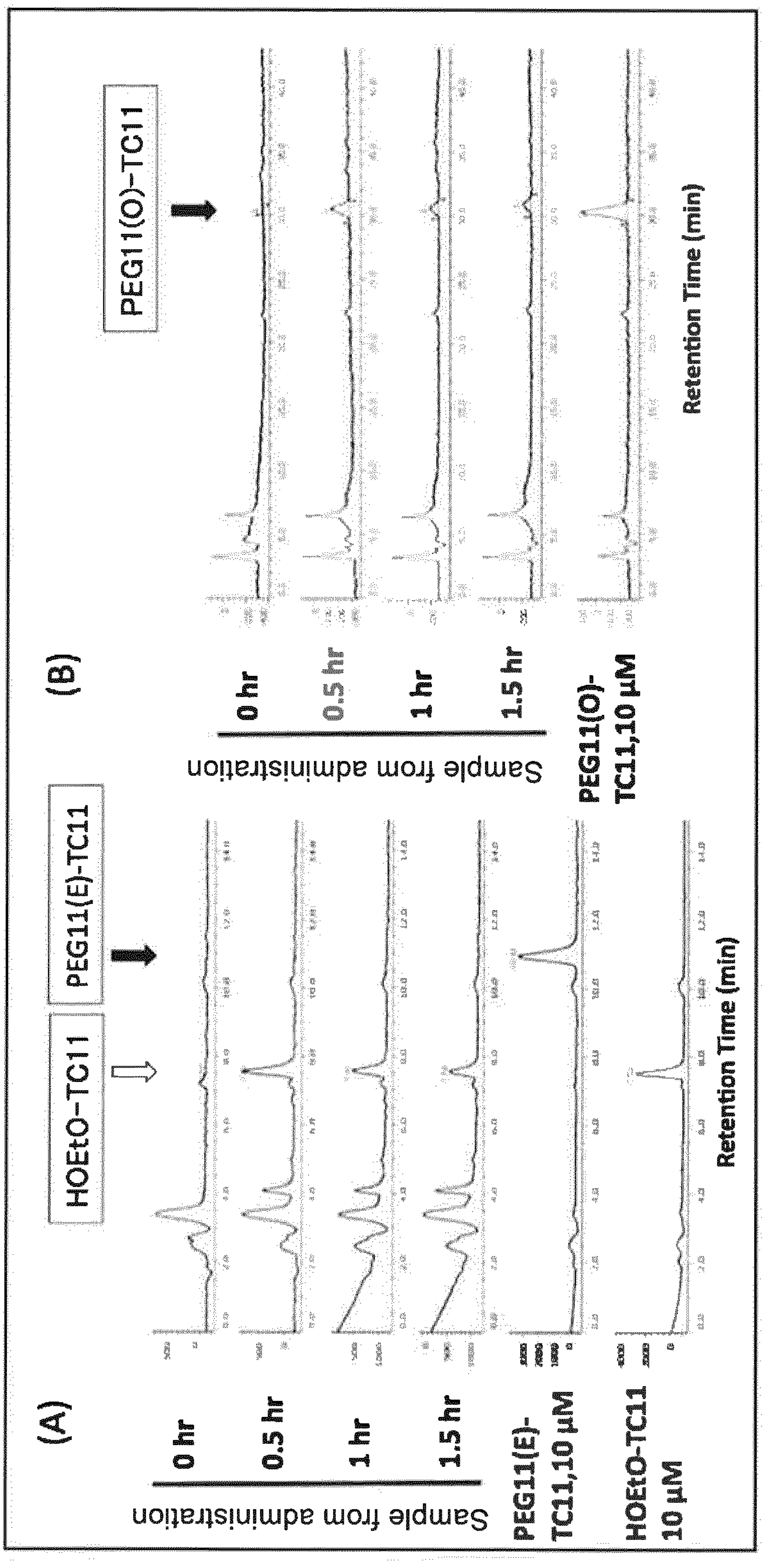
(A)
Sample from administration
0 hr
0.5 hr
1 hr
1.5 hr
PEG11(E)-TC11,10 µM
HOEtO-TC11 10 µM
HOEtO-TC11
PEG11(E)-TC11
Retention Time (min)
(B)
Sample from administration
0 hr
0.5 hr
1 hr
1.5 hr
PEG11(O)-TC11,10 µM
PEG11(O)-TC11
Retention Time (min)

Fig. 2

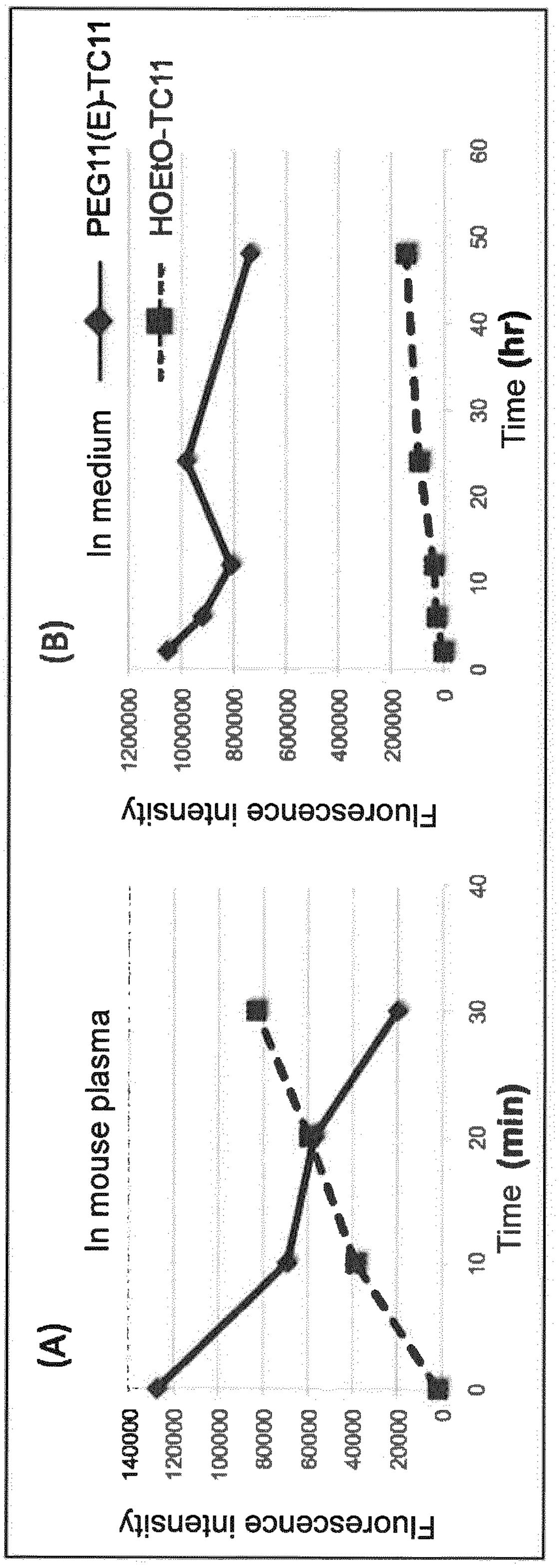
(A)
In mouse plasma
Fluorescence intensity
140000
120000
100000
80000
60000
40000
20000
0
0
10
20
30
40
Time (min)
(B)
In medium
PEG11(E)-TC11
HOEtO-TC11
Fluorescence intensity
1200000
1000000
800000
600000
400000
200000
0
0
10
20
30
40
50
60
Time (hr)

Fig. 3

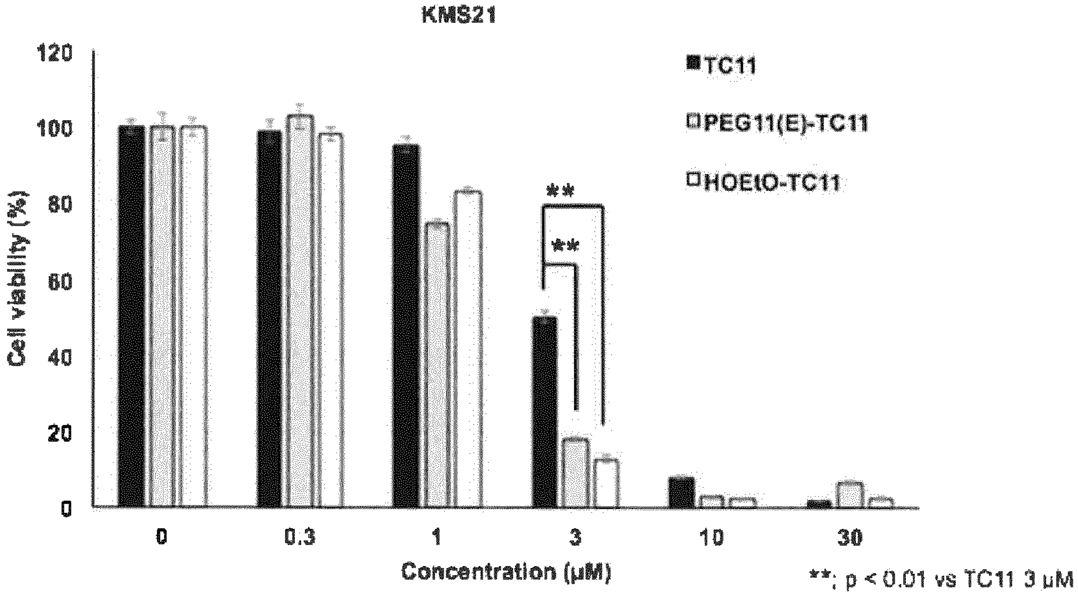
KMS21
120
100
80
60
40
20
0
Cell viability (%)
■TC11
□PEG11(E)-TC11
□HOEtO-TC11
**
**
0
0.3
1
3
10
30
Concentration (μM)
**; p < 0.01 vs TC11 3 μM

Fig. 4

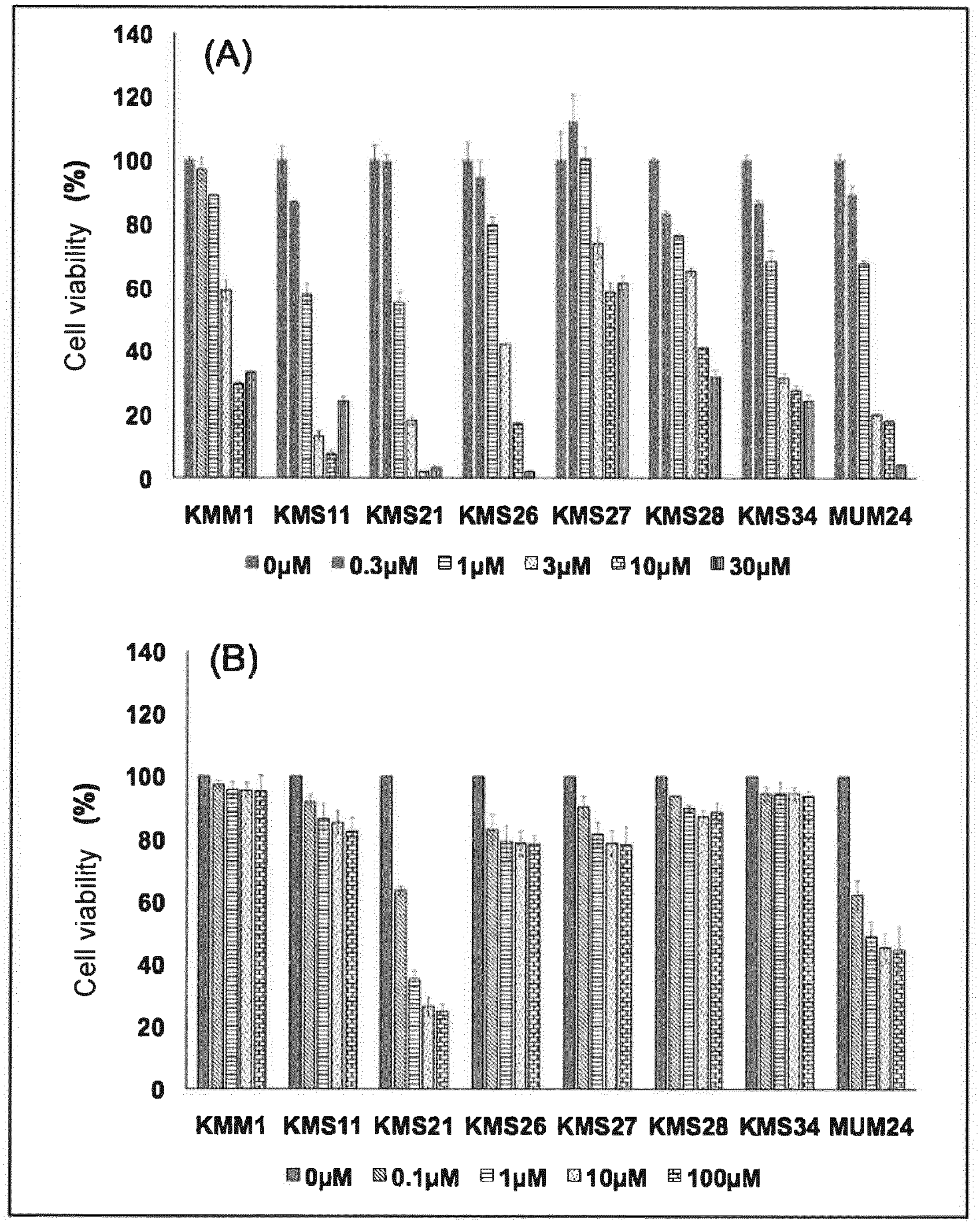
(A)
Cell viability (%)
140
120
100
80
60
40
20
0
KMM1 KMS11 KMS21 KMS26 KMS27 KMS28 KMS34 MUM24
0μM 0.3μM 1μM 3μM 10μM 30μM
(B)
Cell viability (%)
140
120
100
80
60
40
20
0
KMM1 KMS11 KMS21 KMS26 KMS27 KMS28 KMS34 MUM24
0μM 0.1μM 1μM 10μM 100μM

Fig. 5

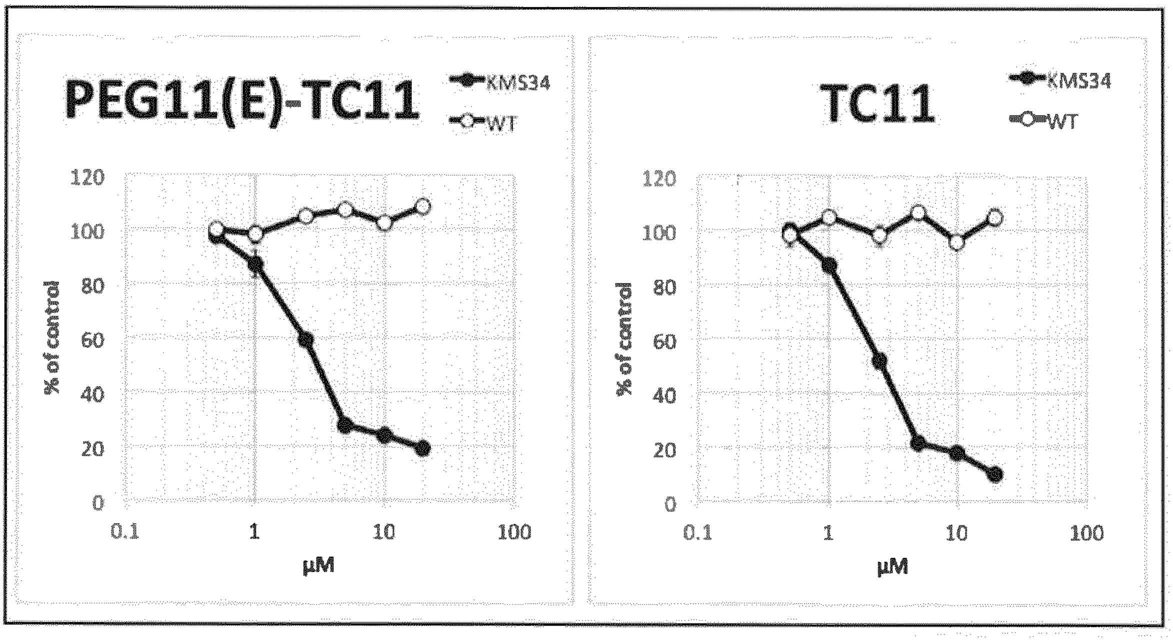
PEG11(E)-TC11
KMS34
WT
120
100
80
60
40
20
0
% of control
0.1
1
10
100
µM
TC11
KMS34
WT
120
100
80
60
40
20
0
% of control
0.1
1
10
100
µM

Fig. 6

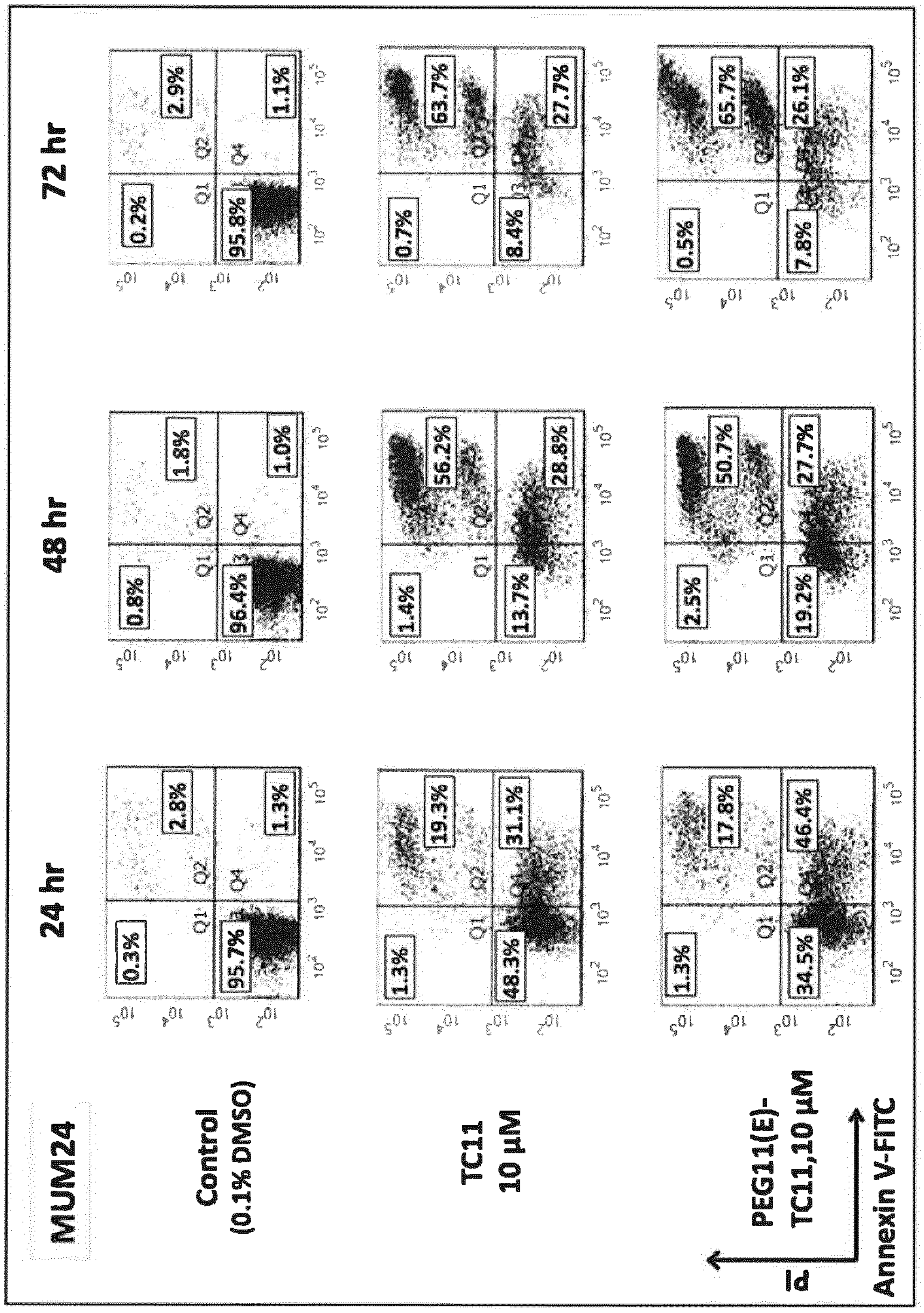
MUM24
24 hr
48 hr
72 hr
Control
(0.1% DMSO)
TC11
10 µM
PEG11(E)-
TC11,10 µM
PI
Annexin V-FITC
0.3%
2.8%
95.7%
1.3%
0.8%
1.8%
96.4%
1.0%
0.2%
2.9%
95.8%
1.1%
1.3%
19.3%
48.3%
31.1%
1.4%
56.2%
13.7%
28.8%
0.7%
63.7%
8.4%
27.7%
1.3%
17.8%
34.5%
46.4%
2.5%
50.7%
19.2%
27.7%
0.5%
65.7%
7.8%
26.1%

Fig. 7

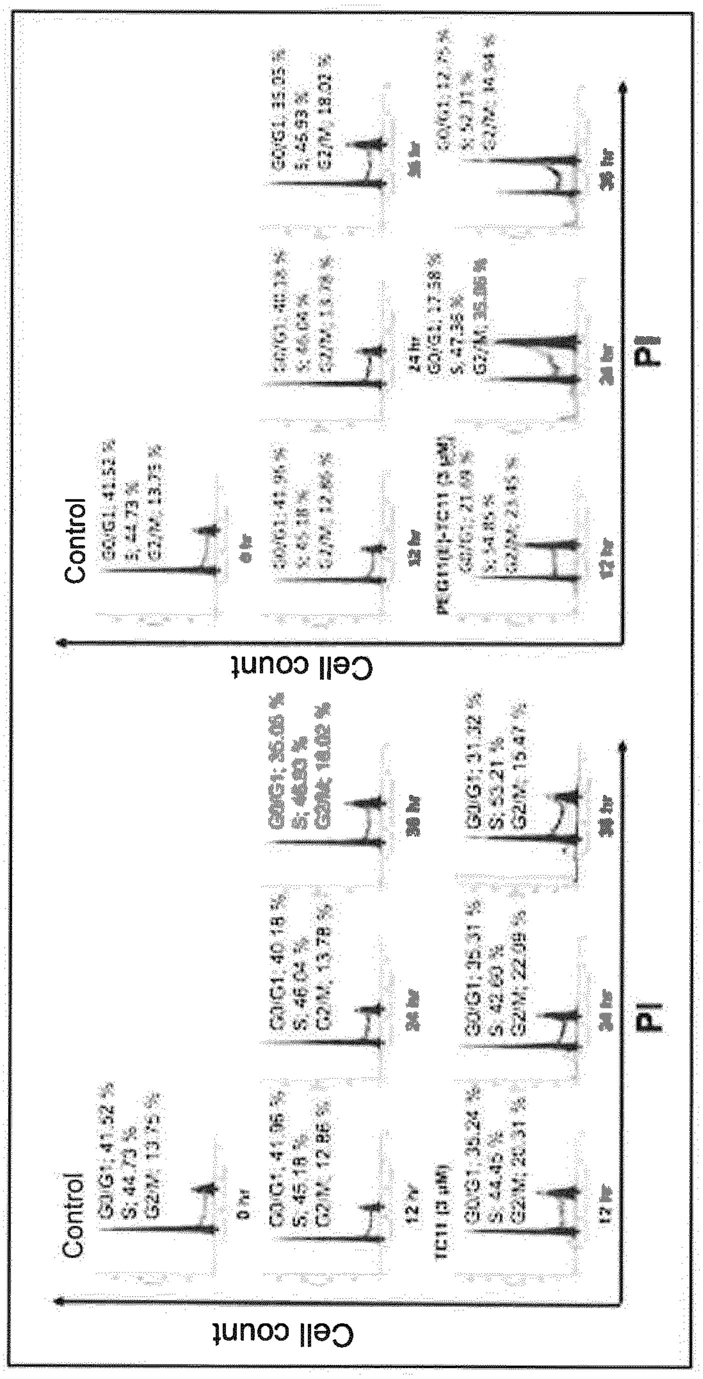


Fig. 8

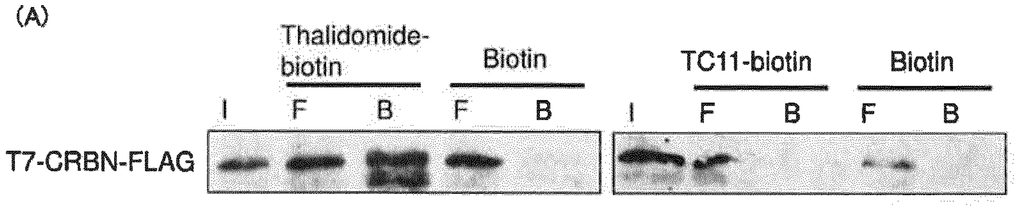
(A)
Thalidomide-biotin
Biotin
TC11-biotin
Biotin
I F B F B I F B F B
T7-CRBN-FLAG

Fig. 9A

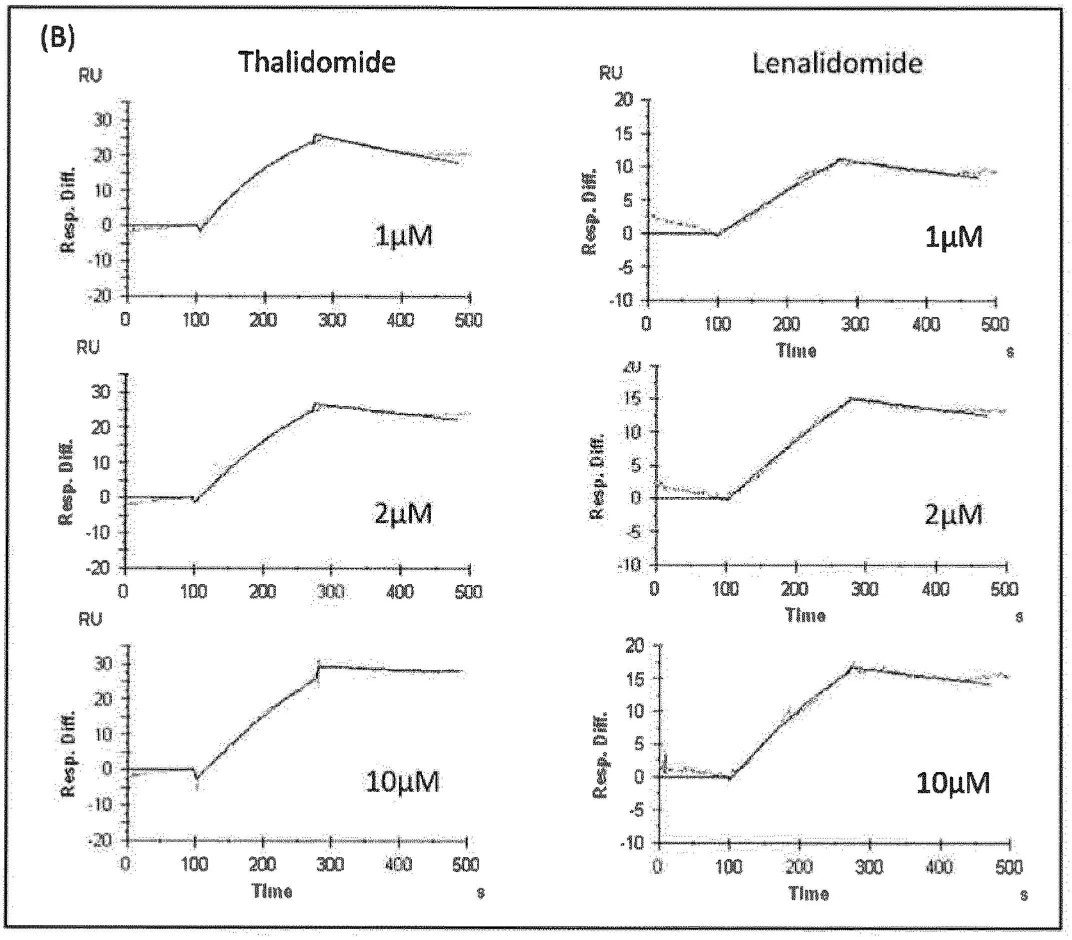
(B)
Thalidomide
Lenalidomide
RU
Resp. Diff.
Time
s
1μM
2μM
10μM

Fig. 9B

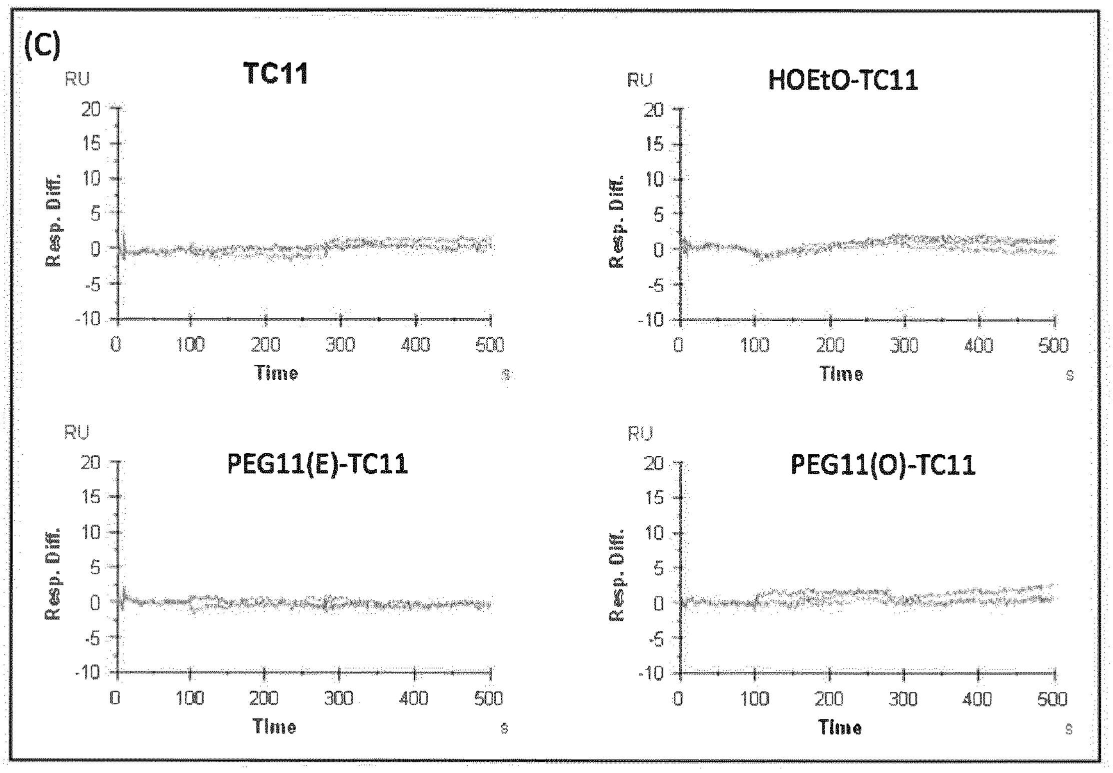
(C)
TC11
HOEtO-TC11
PEG11(E)-TC11
PEG11(O)-TC11
RU
Resp. Diff.
20
15
10
5
0
-5
-10
0
100
200
300
400
500
Time
s

Fig. 9C

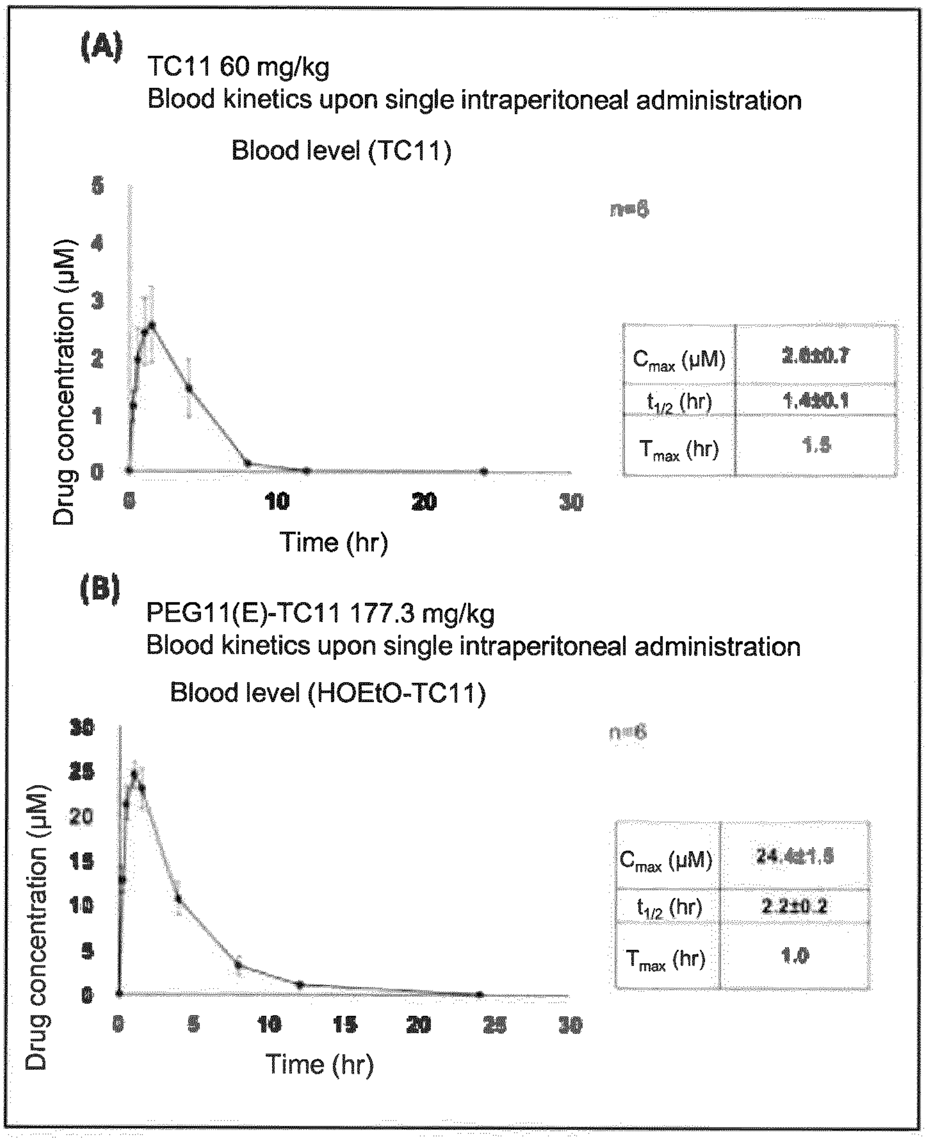
(A)
TC11 60 mg/kg
Blood kinetics upon single intraperitoneal administration
Blood level (TC11)
n=6
Drug concentration (μM)
0
1
2
3
4
5
0
10
20
30
Time (hr)
$C_{max}$ (μM) 2.6±0.7
$t_{1/2}$ (hr) 1.4±0.1
$T_{max}$ (hr) 1.5
(B)
PEG11(E)-TC11 177.3 mg/kg
Blood kinetics upon single intraperitoneal administration
Blood level (HOEtO-TC11)
n=6
Drug concentration (μM)
0
5
10
15
20
25
30
0
5
10
15
20
25
30
Time (hr)
$C_{max}$ (μM) 24.4±1.5
$t_{1/2}$ (hr) 2.2±0.2
$T_{max}$ (hr) 1.0

Fig. 10

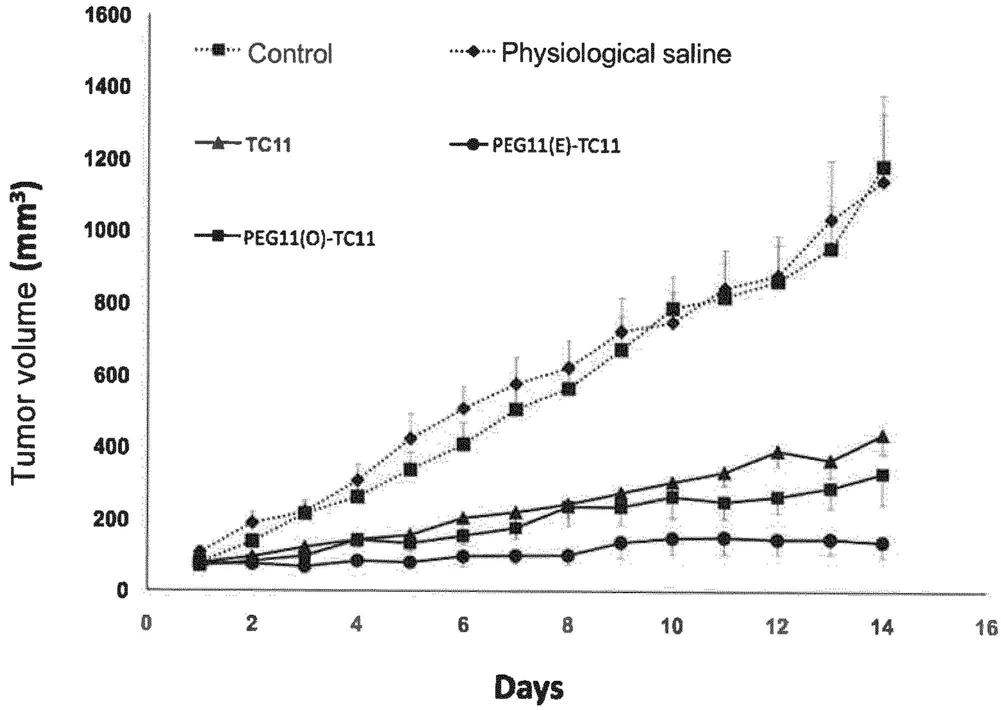

Control
Physiological saline
TC11
PEG11(E)-TC11
PEG11(O)-TC11
Tumor volume (mm³)
Days
0
200
400
600
800
1000
1200
1400
1600
0
2
4
6
8
10
12
14
16


Fig. 11

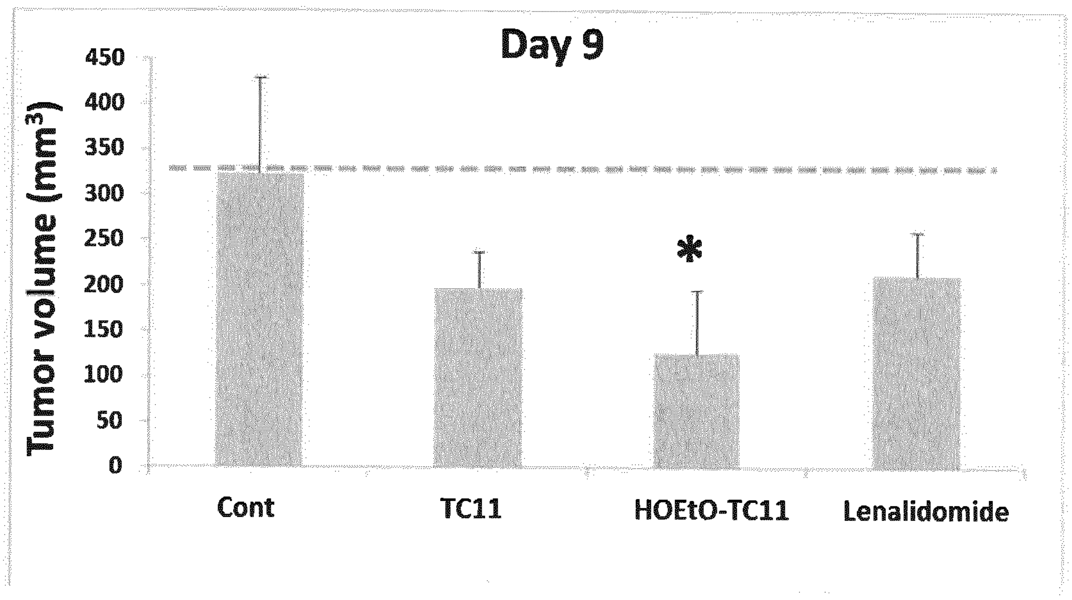

Day 9
Tumor volume (mm³)
0
50
100
150
200
250
300
350
400
450
*
Cont
TC11
HOEtO-TC11
Lenalidomide


Fig. 12

## INTERNATIONAL SEARCH REPORT

International application No.
PCT/JP2017/037906

A. CLASSIFICATION OF SUBJECT MATTER
Int. Cl. C07D209/48(2006.01)i, A61K31/4035(2006.01)i, A61P35/00(2006.01)i

According to International Patent Classification (IPC) or to both national classification and IPC

B. FIELDS SEARCHED

Minimum documentation searched (classification system followed by classification symbols)
Int. Cl. C07D209/48, A61K31/4035, A61P35/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2010/061862 A1 (KEIO UNIVERSITY) 03 June 2010, claims, embodiments (Family: none) | 1-3, 5-9<br>4 |
| Y<br>A | JP 10-109975 A (ISHIHARA SANGYO KAISHA, LTD.) 28 April 1998, claims, compound no. 212 & US 6429212 B1, claims, compound 212 & WO 1998/007421 A1 | 1-3, 5-9<br>4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| | |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2017/037906

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | US 2015/0291562 A1 (ARVINAS, INC.) 15 October 2015, claims, table 1 & JP 2017-513862 A & WO 2015/160845 A2 & AU 2015247817 A & KR 10-2017-0002446 A & CN 106458993 A | 1-3, 5-9<br>4 |
| Y<br>A | WANG, T. et al., Chemistry & Biodiversity, 2009, 6, pp. 466-474, Scheme 3, 13h | 1-3, 5-9<br>4 |
| Y<br>A | LU, J., et al., Chemistry & Biology, 2015, 22, pp. 755-763, fig. 2A | 1-3, 5-9<br>4 |
| A | PASUT, G. et al., Advanced Drug Delivery Reviews, 2009, 61, pp. 1177-1188, whole document | 1-9 |
| A | JP 2013-521265 A (UNIVERSITE JOSEPH FOURIER) 10 June 2013, compound 37 & US 2013/0096083 A1, compound 37 & WO 2011/107709 A1 & FR 2956816 A1 | 1-9 |
| A | WO 2008/047831 A1 (KYOWA HAKKO KIRIN CO., LTD.) 24 April 2008, compounds 151, 219 & EP 2108642 A1, compound 151, 21g | 1-9 |
| A | JP 2010-508278 A (SCHERING CORP.) 18 March 2010, claims, compound 206 & US 2010/0130465 A1, claims, compound 206 & WO 2008/054749 A1 & KR 10-2009-0082440 A & CN 101568527 A | 1-9 |
| A | WALLER, C. L. et al., Journal of Pharmaceutical Sciences, 1994, 83(4), pp. 571-576, Scheme 2 | 1-9 |
| A | BAILLEUX, V., et al., Epilepsia, 1995, 36(6), pp. 559-565, fig. 1 | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010061862 A1
- JP 10109975 A


### Non-patent literature cited in the description


- **SINGHAL, S. et al.** *N. Engl. J. Med.,* 1999, vol. 341, 1565-1571
- **HATTORI, Y. et al.** *Cancer Sci.,* 2008, vol. 99, 1243-1250
- **ITO, T. et al.** *Science,* 2010, vol. 327, 1345-1350
- **DIMOPOULOS, M. et al.** *N. Engl. J. Med.,* 2007, vol. 357, 2123-2132
- **WEBER, DM. et al.** *N. Engl. J. Med.,* 2007, vol. 357, 2133-2142
- **CHAMBERLAIN, PP. et al.** *Nature Struct. Mol. Biol.,* 2014, vol. 21, 803-809
- **AVET-LOISEAU, H. et al.** *Blood,* 2007, vol. 109, 3489-3496
- **AVET-LOISEAU, H. et al.** *Leukemia,* 2010, vol. 24, 623-628
- **KLEIN, U. et al.** *Cancer,* 2011, vol. 117, 2136-2144
- **JAGANNATH, S. et al.** *Leukemia,* 2007, vol. 21, 151-157
- **SIEGEL, DS. et al.** *Blood,* 2012, vol. 120, 2817-2825
- **LOKHORST, HM. et al.** *New Engl. J. Med.,* 2015, vol. 373, 1207-1219
- **SHIHEIDO, H. et al.** *PLoS One,* 2012, vol. 7, e38878
- **SHIBATA, Y. et al.** *Chem. Pharm. Bull.,* 1996, vol. 44, 156-162
- **NOGUCHI, T. et al.** *Bioorg. Med. Chem. Lett.,* 2005, vol. 15, 5509-5513
- **MATSUSHITA, M. et al.** *PLoS One,* 2015, vol. 10, e0116135
- **PASUT, G. ; VERONESE, FM.** *Adv. Drug Del. Rev.,* 2009, vol. 61, 1177-1188
- **OTSUKI, T. et al.** *Int. J. Oncol.,* 1999, vol. 15, 1205-1212
- **HATTORI, Y. et al.** *Blood Cancer J.,* 2013, vol. 3, e115
- **CHANG, H.** *Blood,* 2005, vol. 105, 358-360
- **MOSMAN, T.** *J. Immunol. Methods,* 1983, vol. 65, 55-63
- **COOK, JA. ; MITCHELL, JB.** *Anal. Biochem.,* 1984, vol. 179, 1-7
- **CHAMBERLAIN, PP et al.** *Nature Struct. Mol. Biol.,* 2014, vol. 21, 803-810